# EUROPEAN PATENT APPLICATION

(11) **EP 4 520 764 A1**
(43) Date of publication of application: **12.03.2025**
(21) Application number: 23799139.3
(22) Date of filing: 22.03.2023
(51) Int. Cl.: C07J 43/00, C25B 3/05, C07J 51/00

(54) **METHOD FOR PREPARING ABIRATERONE ACETATE AND INTERMEDIATE THEREOF**

(30) Priority: 06.05.2022 CN 202210488520; 03.08.2022 CN 202210925624; 13.03.2023 CN 202310235319; 13.03.2023 CN 202310235429
(71) Applicant: Aurisco Pharmaceutical co., Ltd., Zhejiang 317200 (CN)
(72) Inventor: XIE, Xiaoqiang, Tiantai Taizhou, Zhejiang 317200 (CN); CHU, Dingjun, Tiantai Taizhou, Zhejiang 317200 (CN); JIANG, Qianqian, Tiantai Taizhou, Zhejiang 317200 (CN); JIN, Jian, Tiantai Taizhou, Zhejiang 317200 (CN)
(74) Representative: Petraz, Gilberto Luigi
(86) International application number: PCT/CN2023/083006
(87) International publication number: WO 2023/213151

(57) **Abstract**

The present invention provides a method for preparing abiraterone acetate and an intermediate thereof. The method particularly relates to the steps of: in an organic solvent, in the presence of a metal catalyst, reacting a 3-site protected 17-hydroxy ester androst-5,16-diene -3β-hydroxy (ester) with a 3-substituted pyridine derivative to obtain abiraterone acetate or the intermediate thereof. The method of the present invention is low in cost, high in yield, simple to operate, and suitable for large-scale industrial production.

## Description

### TECHNICAL FIELD

The present invention relates to the technical field of medicinal chemistry, specifically to a method for preparing abiraterone acetate and intermediate thereof.

### BACKGROUND

Prostate cancer (PCA) refers to an epithelial malignant tumor that occurs in the prostate gland, and is the second most common malignant tumor in men worldwide after lung cancer. It ranks sixth in mortality rate, with approximately 1/9 of men diagnosed with prostate cancer in their lifetime, hence it is known as the "male killer". At present, the global incidence of prostate cancer is increasing. In 2020, there was about 1.5 million new cases worldwide, accounting for 15% of new male tumor cases. It is estimated that the number of prostate cancer patients worldwide will reach 11 million by 2022.

Abiraterone acetate is a prodrug of abiraterone, which rapidly converts into abiraterone in the body. Abiraterone is a selective and irreversible steroid inhibitor of CYP17 (17α-hydroxylase and C₁7,20 lyase), which inhibits enzyme activity to prevent the synthesis of testosterone in testes, renal glands, and tumors. This product was developed by Johnson&Johnson and was first approved by the US FDA on April 28, 2011. It is used in combination with prednisone or prednisolone to treat castration resistant metastatic prostate cancer (mCRPC) in patients who have failed in androgen castration therapy and docetaxel chemotherapy. Later, on December 10, 2012, it was approved to expand the indication population to treat castration resistant advanced metastatic prostate cancer.

There are currently two main methods for preparing abiraterone acetate:
1. The synthesis method reported in WO9509178: dehydroepiandrosterone (DHEA) is used as the raw material, and reacts with hydrazine hydrate to obtain a hydrazone under the catalysis of hydrazine sulfate, and then undergoes an iodination reaction with iodine under the catalysis of tetramethylguanidine (TMG) to obtain vinyl iodide. Then, the iodide undergoes a coupling reaction with diethyl(3-pyridyl) borane under the catalysis of bis (triphenylphosphine) palladium chloride to obtain abiraterone, and finally the 3-site hydroxy is acetylated to obtain abiraterone acetate.

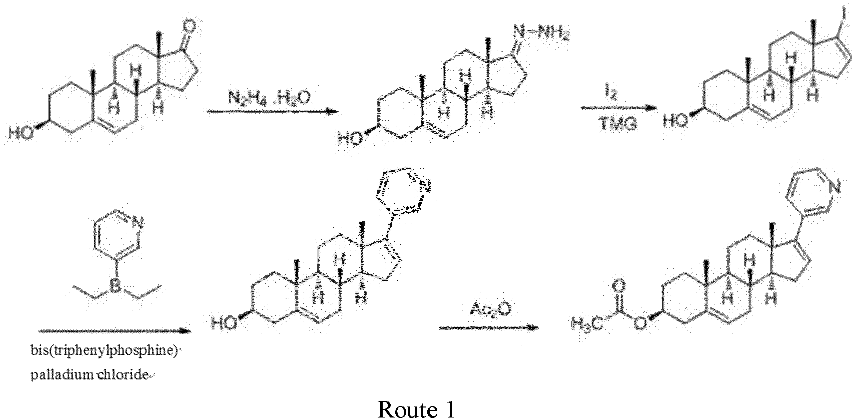

The reaction of the first step in this route requires 5 days, and the reaction of the third step requires 4 days. The production cycle is too long, and the total yield is only 36.9%. The process also requires the use of odorous reagents such as hydrazine hydrate, iodine, and tetramethylguanidine, which causes significant environmental pollution, therefore it is not suitable for industrial production.

2. The synthesis method reported in WO2006021777: dehydroepiandrosterone acetate is used as raw material, and under the catalysis of bases, such as triethylamine, it reacts with trifluoromethanesulfonic anhydride to prepare trifluoromethanesulfonyl derivatives. Then, it couples with diethyl (3-pyridyl) borane under the catalysis of bis (triphenylphosphine) palladium chloride. For the purpose of purification, it forms a salt with methanesulfonic acid to obtain the methanesulfonate salt of abiraterone acetate, with a purity of 96.4% and a total yield of 32.8%. Due to the high price of trifluoromethanesulfonic anhydride, the production cost of this synthesis method is high. Moreover, trifluoromethanesulfonic anhydride has strong hygroscopicity and corrosiveness, the risk of use is high. During the reaction process, 3-dehydroabiraterone is also obtaind, which is difficult to remove by recrystallization and generally needs to be removed by column chromatography.

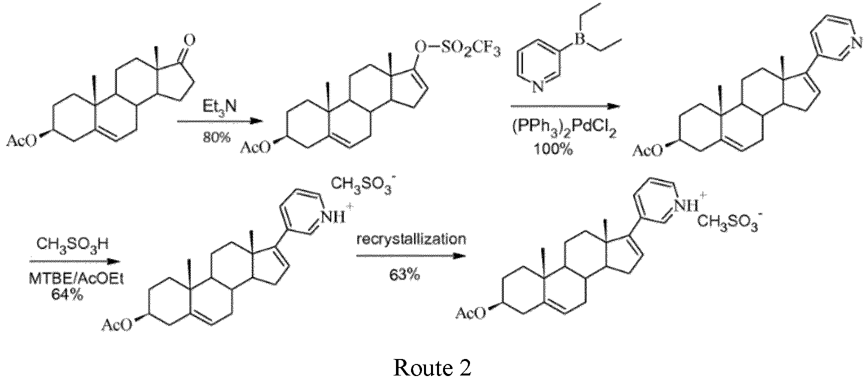

Two preparation methods without using diethyl (3-pyridyl) borane are disclosed in CN103864878A, as shown in routes 3 and 4:

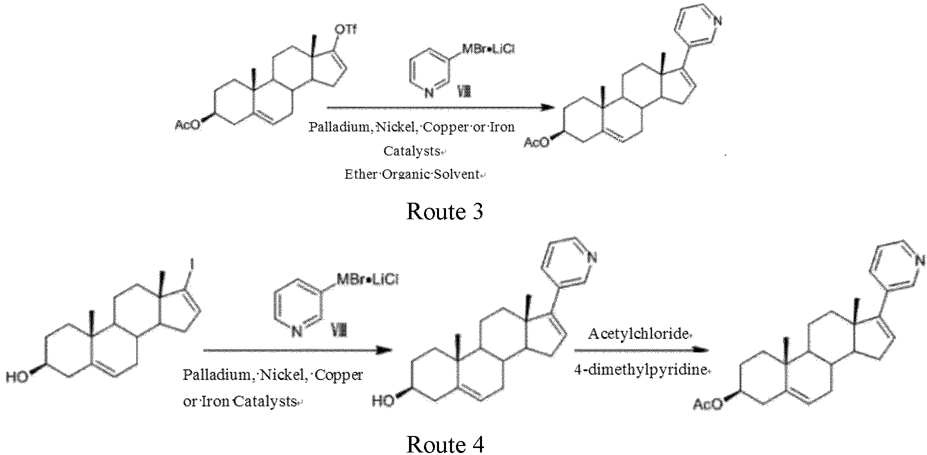

The raw materials for route 3 are the same as route 2, and the trifluoromethanesulfonic anhydride used is expensive. During the reaction process, 3-dehydroabiraterone is also obtaind, which is difficult to remove by recrystallization and generally needs to be removed by column chromatography. The preparation of iodide raw materials in route 4 also has drawbacks such as long reaction time and environmental pollution as in route 1.

Therefore, it is still necessary in this field to find new methods for preparing abiraterone acetate that are low-cost, high-yield, easy to operate, and suitable for industrial production.

### SUMMARY OF THE INVENTION

In response to the above-mentioned problems in the prior art, the purpose of the present invention is to provide a new method for preparing abiraterone acetate and an intermediate thereof, that is easy to operate, high in safety, low in cost, high in yield, and suitable for industrial production.

The first aspect of the present invention provides a method for preparing abiraterone acetate or an intermediate thereof, and the method comprises the steps of: (i) in an organic solvent, in the presence of a metal catalyst, ligand, and reducing agent, reacting a compound of formula II with a compound of formula III to obtain a compound of formula I;
the reaction formula is as follows: 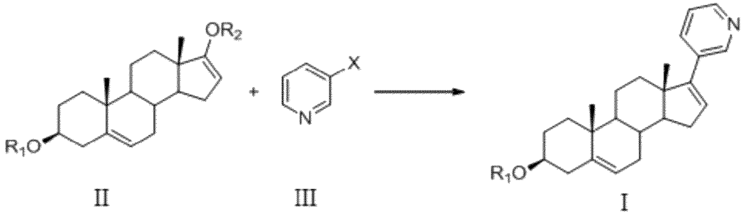
R₁ is selected from the group consisting of: hydrogen, C₁-C₆ alkyl, phenyl, benzyl, C₁-C₆ acyl, substituted or unsubstituted benzoyl, methylsulfonyl, substituted or unsubstituted benzenesulfonyl, or C₁-C₂ trialkylsilyl;
R₂ is selected from the group consisting of: C₁-C₆ acyl, substituted or unsubstituted benzoyl, methylsulfonyl, substituted or unsubstituted benzenesulfonyl, dimethylamino acyl or diethylaminoacyl, or diphenylphosphinyl acyl; and
X is fluorine, chlorine, bromine or iodine;
unless otherwise specified, the "substituted" refers to that one or more hydrogen atoms on a group is independently substituted by a group selected from the group consisting of: halogen, C₁-C₄ alkyl, C₁-C₄ haloalkyl, ether, and nitro;
the metal catalyst is selected from the group consisting of: palladium salt, copper salt, iron salt, cobalt salt, nickel salt, or combinations thereof; and
the ligand is selected from the group consisting of: phosphorus-containing ligand, amino acid ligand, nitrogen-containing ligand, or combinations thereof.

In another preferred embodiment, R₁ and R₂ are the same or different.

In another preferred embodiment, R₁ is acetyl.

In another preferred embodiment, in step (i), the molar ratio of the compound of formula II to the compound of formula III is 1: 1~4, preferably 1: 1.5~3, such as 1: 2 or 1: 3.

In another preferred embodiment, in step (i), the molar ratio of the compound of formula II to the metal catalyst is 1: 0.005~0.3; preferably 1: 0.01~0.2, such as 1: 0.03, 1: 0.05, 1: 0.07 or 0.1.

In another preferred embodiment, in step (i), the molar ratio of the compound of formula II to the ligand is 1: 0.005~0.3; preferably 1: 0.01~0.2, such as 1: 0.03, 1: 0.05; 1: 0.07 or 0.1.

In another preferred embodiment, in step (i), the molar ratio of the compound of formula II to the reducing agent is 1: 1~4, preferably 1: 1.5~3, such as 1: 2 or 1: 3.

In another preferred embodiment, the metal catalyst is selected from the group consisting of: bis triphenylphosphine palladium chloride, palladium acetate, palladium chloride, cuprous iodide, copper acetate, ferric chloride, acetylacetonate iron, cobalt chloride, acetylacetonate cobalt, nickel acetate, nickel chloride, tricyclohexylphosphine nickel chloride, or combinations thereof.

In another preferred embodiment, the ligand is selected from the group consisting of: triphenylphosphine and derivatives thereof, tricyclohexylphosphine and derivatives thereof, L-proline and derivatives thereof, pyridine and derivatives thereof, 2,2-biphyridine and derivatives thereof, 1,10-phenanthroline and derivatives thereof, or combinations thereof.

In another preferred embodiment, the reducing agent is selected from the group consisting of: diboride reagent, zinc powder, copper powder, iron powder, magnesium powder, manganese powder, tin powder, samarium powder, indium powder, or combinations thereof.

In another preferred embodiment, the organic solvent is selected from the group consisting of: tetrahydrofuran, toluene, acetonitrile, N,N-dimethylformamide, N,N-dimethylacetamide, N-methylpyrrolidone, dimethyl sulfoxide, or combinations thereof.

In another preferred embodiment, in step (i), the reaction temperature is 20-120 °C, preferably 40-100 °C.

In another preferred embodiment, the method further comprises the steps of:
(i-a) reacting the compound of formula IV with an acylation reagent to obtain the compound of formula II;
the reaction formula is as follows: 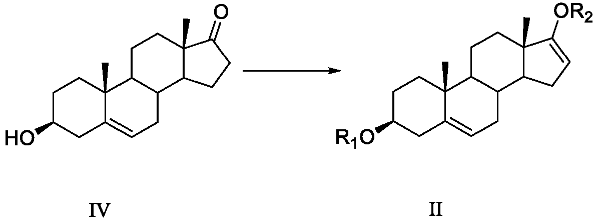
wherein, the acylation reagent used to obtain R₁ is selected from the group consisting of: R₁-halogen, R₁-O-R₁, isopropenyl acetate, or combinations thereof; and
the acylation reagent used to obtain R₂ is selected from the group consisting of: R₂-halogen, R₂-O-R₂, isopropenyl acetate, or combinations thereof.

In another preferred embodiment, the acylation reagent used to obtain R₁ is selected from the group consisting of: C₁-C₆ alkyl acyl chlorides, substituted or unsubstituted benzoic anhydrides, isopropenyl acetate, trimethylchlorosilane, or combinations thereof.

In another preferred embodiment, the acylation reagent used to obtain R₂ is selected from the group consisting of: isopropenyl acetate, substituted or unsubstituted benzoic anhydride, substituted or unsubstituted benzenesulfonic anhydride, or combinations thereof.

The present invention further provides a method for preparing abiraterone acetate, the method comprises the steps of:
(i) preparing the compound of formula I by the above method;
(ii) in an organic solvent, the compound of formula I undergoes a hydrolysis reaction with sodium hydroxide solution to obtain abiraterone; and
(iii) abiraterone undergoes an esterification reaction with acetic anhydride to obtain abiraterone acetate.

In another preferred embodiment, in step (ii), the organic solvent is selected from methanol, ethanol, isopropanol, or combinations thereof.

The second aspect of the present invention provides a method (electrochemical preparation method) for preparing abiraterone acetate or an intermediate thereof, wherein the method comprises the steps of: (1) in a solvent, in the presence of a metal catalyst, ligand, and electrolyte, under the constant current, reacting a compound of formula II with a compound of formula III to obtain a compound of formula I, and the reaction formula is as follows: 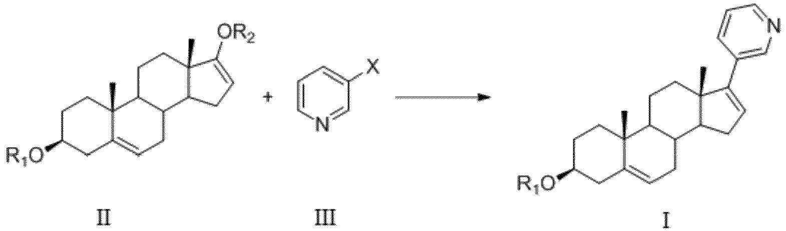
wherein, R₁ is selected from hydrogen, C₁-C₆ alkyl, phenyl, benzyl, C₁-C₆ acyl, substituted or unsubstituted benzoyl, methylsulfonyl, substituted or unsubstituted benzenesulfonyl, or C₁-C₂ trialkylsilyl or dialkylarylsilyl, and the alkyl in dialkylarylsilyl is a C₁-C₃ alkyl;
R₂ is selected from C₁-C₆ acyl, substituted or unsubstituted benzoyl, methylsulfonyl, substituted or unsubstituted benzenesulfonyl, dimethylamino acyl, diethylamino acyl, or diphenylphosphinyl acyl;
the term "substituted" refers to that one or more hydrogen atoms on a group are independently substituted by a group selected from the group consisting of:
   halogen, C₁-C₄ alkyl or C₁-C₄ haloalkyl, ether, nitro;
   X is fluorine, chlorine, bromine or iodine;
   the metal catalyst is selected from palladium salt, copper salt, cobalt salt, nickel salt, or combinations thereof; and
   the ligand is selected from phosphorus-containing ligands, amino acid-containing ligands, pyridine-containing ligands, or combinations thereof.

In another preferred embodiment, R₁ is acetyl, and the compound of formula I is abiraterone acetate.

In another preferred embodiment, in step (1), the molar ratio of the compound offormula II to the compound of formula III is 1: 1~4. In another more preferred embodiment, the molar ratio of the compound of formula II to the compound of formula III is 1: 1~3. In another more preferred embodiment, the molar ratio of the compound of formula II to the compound of formula III is 1: 1.5~2.2, such as 1: 1.8 or 1: 2.5;

In another preferred embodiment, in step (1), the molar ratio of the compound of formula II to the metal catalyst is 1: 0.005~0.2. In another more preferred embodiment, the molar ratio of the compound of formula II to the metal catalyst is 1: 0.01~0.1. In another more preferred embodiment, the molar ratio of the compound of formula II to the metal catalyst is 1: 0.03~0.07, such as 1: 0.04, 1: 0.005 or 1: 0.006;

In another preferred embodiment, in step (1), the molar ratio of the compound of formula II to the ligand is 1: 0.005~0.3. In another more preferred embodiment, the molar ratio of the compound of formula II to the ligand is 1: 0.01~0.2. In another more preferred embodiment, the molar ratio of the compound of formula II to the ligand is 1: 0.05~0.12, such as 1: 0.06, 1: 0.08, 1: 0.10.

In another preferred embodiment, in step (1), the molar volume ratio of electrolyte to reaction solution is 0.1~0.5 mol/L.

In another preferred embodiment, in step (1), the constant current is 0.05A~1.0A. In another more preferred embodiment, the constant current is 0.1A~0.5A.

In another preferred embodiment, in step (1), the current density is 0.01~0.2A/cm². In another more preferred embodiment, the current density is 0.02~0.1A/cm².

In another preferred embodiment, in step (1), the reaction temperature is 0 °C~60 °C. In another more preferred embodiment, the reaction temperature is 10 °C~50 °C.

In another preferred embodiment, the metal catalyst is selected from the group consisting of bis triphenylphosphine palladium chloride, palladium acetate, palladium chloride, palladium trifluoromethanesulfonate, cuprous iodide, copper acetate, copper chloride, cobalt chloride, cobalt acetylacetonate, cobalt acetate, cobalt sulfate, nickel acetate, tricyclohexylphosphine nickel chloride, or combinations thereof.

In another preferred embodiment, the ligand is selected from the group consisting of triphenylphosphine, tricyclohexylphosphine, tri tert-butylphosphine, L-proline, alanine, methionine, pyridine, 2,2-biphyridine, 1,10-phenanthroline, or derivatives thereof.

In another preferred embodiment, the electrolyte is selected from the group consisting of tetraethyl ammonium perchlorate, tetraethyl ammonium p-toluenesulfonate, tetrabutyl ammonium acetate, tetrabutyl ammonium hexafluorophosphate, tetrabutyl ammonium tetrafluoroborate, tetraethyl ammonium tetrafluoroborate, tetraethyl ammonium hexafluorophosphate, or combinations thereof.

In another preferred embodiment, the solvent used in the reaction is selected from the group consisting of acetonitrile, N,N-dimethylformamide, N,N-dimethylacetamide, N-methylpyrrolidone, dimethyl sulfoxide, or combinations thereof.

In another preferred embodiment, the anode electrode used in the reaction is iron, zinc, magnesium, nickel, aluminum, or combinations thereof, and the cathode electrode is platinum.

In another preferred embodiment, R₂ in compound of formula II is acetyl, X in compound of formula III is bromine, the metal catalyst is PdCl₂(PPh₃)₂, and the ligand is bipyridine.

In another preferred embodiment, R₂ in compound of formula II is acetyl, X in compound of formula III is iodine, the metal catalyst is nickel acetate, and the ligand is tricyclohexylphosphine.

In another preferred embodiment, R₂ in compound of formula II is p-toluenesulfonyl, X in compound of formula III is chlorine, the metal catalyst is cuprous iodide, and the ligand is L-proline, or
in another preferred embodiment, R₂ in compound of formula II is benzoyl, X in compound of formula III is fluorine, the metal catalyst is nickel chloride, and the ligand is 1,10-phenanthroline.

In another preferred embodiment, the step (1) comprises: a solvent, a compound of formula II, a compound of formula III, a metal catalyst, a ligand, and an electrolyte are added to the reactor sequentially, then the anode electrode and cathode electrode are fixed to the reactor, the power is turned on, and the reaction is carried out under constant current.

In another preferred embodiment, the method further comprises reacting the compound of formula IV with an acylation reagent to obtain the compound of formula II.

The reaction formula is as follows: 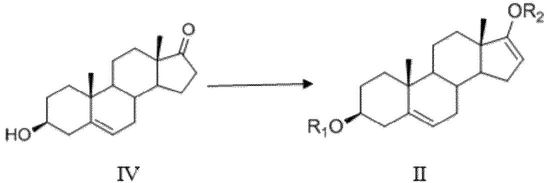
wherein, the acylation reagent used to obtain R₁ is selected from R₁-halogen, R₁-O-R₁, or 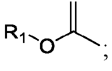 and
the acylation reagent used to obtain R₂ is selected from R₂-halogen, R₂-O-R₂, or

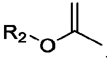

In another preferred embodiment, the acylation reagent used to obtain R₁ is selected from C₁-C₆ alkyl acyl chlorides, substituted or unsubstituted benzoic anhydrides, isopropenyl acetate, or trimethylchlorosilane.

In another preferred embodiment, the acylation reagent used to obtain R₂ is selected from isopropenyl acetate, substituted or unsubstituted benzoic anhydride, or substituted or unsubstituted benzenesulfonic anhydride.

The present invention further provides a method for preparing abiraterone, wherein the method comprises the steps of: (2) the compound of formula I prepared by the above preparation method is subjected to deprotection to obtain abiraterone, and the reaction formula is as follows:

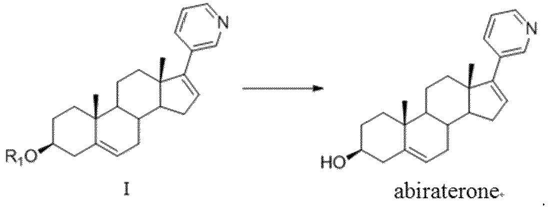

In another preferred embodiment, the deprotection is carried out in a solvent selected from alcohols.

In another preferred embodiment, the alcohol is selected from methanol, ethanol, isopropanol, or combinations thereof.

In another preferred embodiment, the deprotection is carried out in the presence of a base selected from sodium hydroxide and/or potassium hydroxide.

In another preferred embodiment, the deprotection is carried out at 50-100 °C.

The present invention further provides a method for preparing abiraterone acetate, wherein the method comprises the steps of: (3) reacting abiraterone prepared by the above preparation method with acetic anhydride to obtain abiraterone acetate, and the reaction formula is as follows:

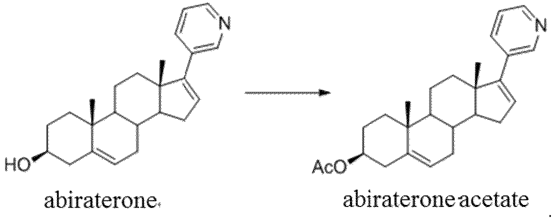

In another preferred embodiment, the solvent used for the reaction between abiraterone and acetic anhydride is selected from dichloromethane.

In another preferred embodiment, the reaction between abiraterone and acetic anhydride is carried out in the presence of a base selected from triethylamine.

In another preferred embodiment, the temperature for the reaction between abiraterone and acetic anhydride is 0~40 °C, more preferably 10~30 °C.

The third aspect of the present invention provides a method for preparing abiraterone acetate or an intermediate thereof, wherein the method comprises the steps of: (1) in a solvent, in the presence of a metal catalyst, ligand, and base, reacting a compound of formula II with a compound of formula III-1 to obtain a compound of formula I.

The reaction formula is as follows: 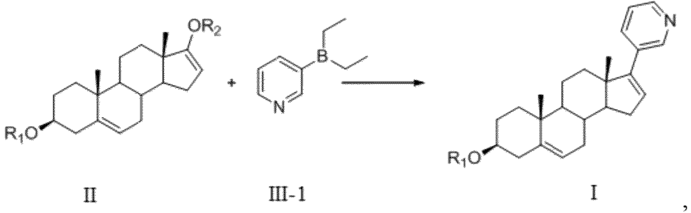
wherein, R₁ is selected from hydrogen, C₁-C₆ alkyl, phenyl, benzyl, C₁-C₆ acyl, substituted or unsubstituted benzoyl, trialkylsilyl or dialkylarylsilyl, and the alkyl in trialkylsilyl and dialkylarylsilyl is C₁-C₃ alkyl,
R₂ is selected from C₁-C₆ acyl, substituted or unsubstituted benzoyl, dimethylamino acyl, diethylamino acyl, or diphenylphosphinyl acyl;
the term "substituted" refers to that one or more hydrogen atoms on a group are independently substituted by a group selected from the group consisting of:
   halogen, C₁-C₄ alkyl, C₁-C₄ haloalkyl, ether, nitro, etc,
   the metal catalyst is selected from palladium salt, copper salt, iron salt, cobalt salt, nickel salt, or combinations thereof;
   the ligand is selected from phosphorus-containing ligands, amino acid ligands, nitrogen-containing ligands, or combinations thereof.

In another preferred embodiment, R₁ is selected from C₁-C₆ acyl, substituted or unsubstituted benzoyl, or trialkylsilyl. In another more preferred embodiment, R₁ is selected from acetyl.

In another preferred embodiment, R₂ is selected from C₁-C₆ acyl, substituted or unsubstituted benzoyl, or dimethylamino acyl. In another more preferred embodiment, R₂ is selected from acetyl.

In another preferred embodiment, in step (1), the molar ratio of the compound of formula II to the compound of formula III-1 is 1: 1~3. In another more preferred embodiment, the molar ratio of the compound of formula II to the compound of formula III-1 is 1: 1~ 1.5.

In another preferred embodiment, in step (1), the molar ratio of the compound of formula II to the metal catalyst is :0.005~0.3. In another more preferred embodiment, the molar ratio of the compound of formula II to the metal catalyst is 1: 0.01~0.2.

In another preferred embodiment, in step (1), the molar ratio of the compound of formula II to the ligand is 1: 0.005~0.3. In another more preferred embodiment, the molar ratio of the compound of formula II to the ligand is 1: 0.01~0.2.

In another preferred embodiment, in step (1), the molar ratio of the compound of formula II to the base is 1: 1~3. In another more preferred embodiment, the molar ratio of the compound of formula II to the base is 1: 1~2.

In another preferred embodiment, the metal catalyst is selected from palladium acetate, palladium chloride, cuprous iodide, copper acetate, ferric chloride, ferric acetylacetonate, cobalt chloride, cobalt acetylacetonate, nickel acetate, nickel chloride, or combinations thereof.

In another preferred embodiment, the ligand is selected from triphenylphosphine, tricyclohexylphosphine, tri tert-butylphosphine, L-proline, alanine, methionine, pyridine, 2,2-bipyridine, 1,10-phenanthroline, or derivatives thereof.

In another preferred embodiment, the base is selected from potassium carbonate, sodium carbonate, potassium phosphate, lithium tert-butoxide, sodium hydroxide, sodium bicarbonate, or combinations thereof.

In another preferred embodiment, the solvent is selected from tetrahydrofuran, toluene, acetonitrile, N,N-dimethylformamide, N,N-dimethylacetamide, N-methylpyrrolidone, dimethyl sulfoxide, or combinations thereof.

In another preferred embodiment, R₂ in the compound of formula II is acetyl, the metal catalyst is palladium chloride, the ligand is triphenylphosphine, and the base is potassium carbonate.

In another preferred embodiment, R₂ in the compound of formula II is propionyl, the metal catalyst is nickel acetate, the ligand is 2-pyridine, and the base is potassium phosphate.

In another preferred embodiment, R₂ in the compound of formula II is N,N-dimethylformamide, the metal catalyst is cuprous iodide, the ligand is 1,10-phenanthroline, and the base is sodium bicarbonate.

In another preferred embodiment, R₂ in the compound of formula II is benzoyl, the metal catalyst is nickel chloride, the ligand is tricyclohexylphosphine, and the base is lithium tert butoxide.

In another preferred embodiment, in step (1), the reaction temperature is 40-130 °C. In another preferred embodiment, the reaction temperature for step (1) is 60-115 °C.

In another preferred embodiment, the preparation method further comprises the following steps:
(1-a) reacting the compound of formula IV with an acylation reagent to obtain the compound of formula II; the reaction formula is as follows: 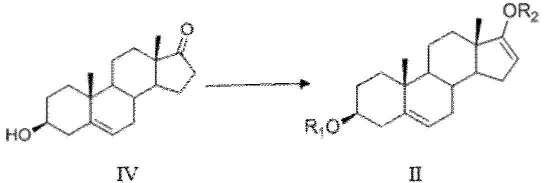
wherein, the acylation reagent used to obtain R₁ is selected from R₁-halogen, R₁-O-R₁ or 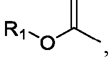 and the acylation reagent used to obtain R₂ is selected from R₂-halogen, R₂-O-R₂ or.

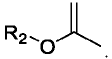

In another preferred embodiment, the acylation reagent used to obtain R₁ is selected from C₁-C₆ alkyl acyl chlorides, substituted or unsubstituted benzoic anhydrides, isopropenyl acetate, or trimethylchlorosilane, and the acylation reagent used to obtain R₂ is selected from isopropenyl acetate, or substituted or unsubstituted benzoic anhydride.

The present invention further provides a method for preparing abiraterone, wherein the method comprises deprotecting the compound of formula I prepared by the above preparation method to obtain abiraterone, and the reaction formula is as follows:

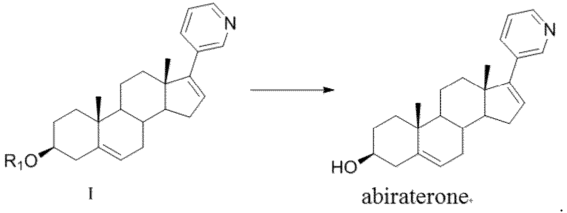

The present invention further provides a method for preparing abiraterone acetate, wherein the method comprises reacting the abiraterone prepared by the above preparation method with acetic anhydride to obtain abiraterone acetate, and the reaction formula is as follows:

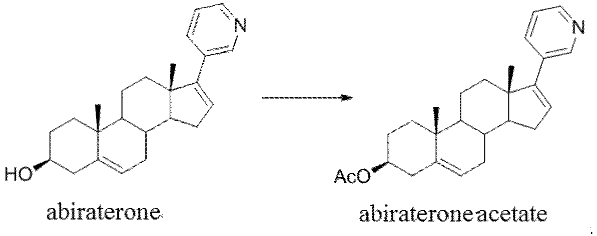

The fourth aspect of the present invention provides a method for preparing abiraterone acetate or an intermediate thereof, wherein the method comprises the steps of: (1) in an organic solvent, in the presence of a metal catalyst, reacting a compound of formula II with a compound of formula III-2 to obtain a compound of formula I;
the reaction formula is as follows: 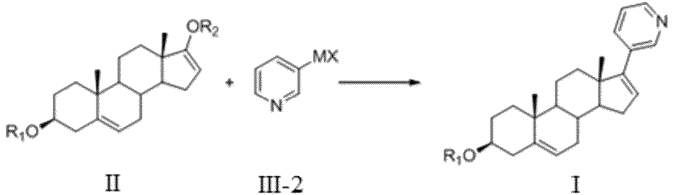
wherein, R₁ is selected from hydrogen, C₁-C₆ alkyl, phenyl, benzyl, C₁-C₆ acyl, substituted or unsubstituted benzoyl, methylsulfonyl, substituted or unsubstituted benzenesulfonyl, trialkylsilyl or dialkylarylsilyl, and the alkyl group in trialkylsilyl and dialkylarylsilyl is C₁-C₃ alkyl,
R₂ is selected from C₁-C₆ acyl, substituted or unsubstituted benzoyl, methylsulfonyl, substituted or unsubstituted benzenesulfonyl, dimethylamino acyl, diethylamino acyl, or diphenylphosphinyl acyl,
the term "substituted" refers to that one or more hydrogen atoms on a group are independently substituted by a group selected from the group consisting of:
   halogen, C₁-C₄ alkyl, C₁-C₄ haloalkyl, ether, nitro, etc,
   M is magnesium, iron, zinc, copper, tin, manganese, bismuth or indium,
   X is fluorine, chlorine, bromine, iodine, or a coordinated anion of M, preferably, the coordinated anion of M is selected from pivalate or acetate,
   the metal catalyst is selected from a palladium salt, copper salt, iron salt, cobalt salt, nickel salt, or combinations thereof.

In another preferred embodiment, there are also ligands present in the reaction system, wherein the ligand is selected from a phosphorus-containing ligand, amino acid ligand, nitrogen-containing ligand, or combinations thereof.

In another preferred embodiment, there are also additives present in the reaction system, wherein the additive is selected from an alkali metal salt.

In another preferred embodiment, there are ligands and additives present in the reaction system, wherein the ligand is selected from a phosphorus-containing ligand, amino acid ligand, nitrogen-containing ligand, or combinations thereof, and the additive is selected from an alkali metal salt.

In another preferred embodiment, R₁ is selected from C₁-C₆ acyl, substituted or unsubstituted benzoyl, or trialkylsilyl, and the alkyl in trialkylsilyl is C₁-C₃ alkyl. In another more preferred embodiment, R₁ is selected from acetyl.

In another preferred embodiment, R₂ is selected from C₁-C₆ acyl, or substituted or unsubstituted benzoyl. In another more preferred embodiment, R₂ is selected from acetyl.

In another preferred embodiment, M is magnesium, zinc, tin, or manganese

In another preferred embodiment, X is bromine or iodine.

In another preferred embodiment, the metal catalyst is selected from palladium chloride, palladium acetate, palladium chloride, cuprous iodide, copper acetate, copper sulfate, ferric chloride, ferrous chloride, ferric acetylacetonate, ferrous acetylacetonate, cobalt chloride, cobalt acetate, acetylacetonate cobalt, nickel chloride, nickel bromide, nickel acetate, tricyclohexylphosphine nickel chloride, or combinations thereof.

In another preferred embodiment, in step (1), the organic solvent is selected from tetrahydrofuran, 2-methyltetrahydrofuran, ethylene glycol dimethyl ether, methyl tert-butyl ether, ether, toluene, or combinations thereof.

In another preferred embodiment, in step (1), the molar ratio of the compound of formula II to the compound of formula III-2 is 1: 1.0~4.0. In another more preferred embodiment, the molar ratio of the compound of formula II to the compound of formula III-2 is 1: 1.0~2.0.

In another preferred embodiment, in step (1), the molar ratio of the compound of formula II to the metal catalyst is 1: 0.005~0.3. In another more preferred embodiment, the molar ratio of the compound of formula II to the metal catalyst is 1: 0.01~0.2.

In another preferred embodiment, in step (1), the temperature for dropwise addition of the compound of III-2 is -30 °C~25 °C, and the reaction temperature is 0~70 °C. In another preferred embodiment, in step (1), the reaction temperature is room temperature ~ 50 °C.

In another preferred embodiment, the ligand is selected from triphenylphosphine, tricyclohexylphosphine, tri tert-butylphosphine, L-proline, alanine, methionine, pyridine, 2,2-bipyridine, 1,10-phenanthroline, or derivatives thereof.

In another preferred embodiment, the molar ratio of the compound of formula II to the ligand is 1: 0.005~0.3. In another preferred embodiment, the molar ratio of the compound of formula II to the ligand is 1: 0.01~0.2.

In another preferred embodiment, the additive is selected from potassium chloride, potassium carbonate, potassium tert-butoxide, potassium bicarbonate, potassium phosphate, potassium acetate, sodium carbonate, sodium bicarbonate, sodium acetate, lithium chloride, lithium carbonate, or combinations thereof.

In another preferred embodiment, the molar ratio of the compound of formula II to the additive is 1: 0.1~3. In another more preferred embodiment, the molar ratio of the compound of formula II to the additive is 1: 1~2.

In another preferred embodiment, in the compound of formula II, R₂ is acetyl, the compound of III-2 is 3-pyridyl magnesium bromide, the metal catalyst is PdCl₂, the ligand is triphenylphosphine, and the additive is sodium acetate.

In another preferred embodiment, in the compound of formula II, R₂ is acetyl, the compound of III-2 is 3-pyridyl zinc bromide, the metal catalyst is anhydrous ferric chloride, the ligand is tetramethylethylenediamine (TMEDA), and the additive is potassium acetate.

In another preferred embodiment, in the compound of formula II, R₂ is p-phenylsulfonyl, the compound of III-2 is 3-pyridyl tin bromide, the metal catalyst is nickel acetate, the ligand is tricyclohexylphosphine, and the additive is sodium carbonate.

In another preferred embodiment, in the compound of formula II, R₂ is benzoyl, the compound of III-2 is 3-pyridy manganese bromide, the metal catalyst is cuprous iodide, the ligand is L-proline, and the additive is anhydrous potassium phosphate.

In another preferred embodiment, the above preparation method further comprises the following steps:
(1-a) reacting the compound of formula IV with an acylation reagent to obtain the compound of formula II;
the reaction formula is as follows: 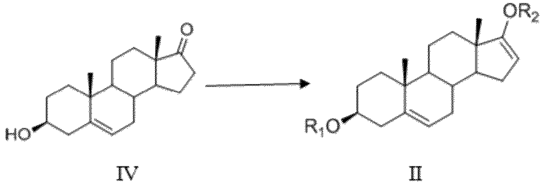
wherein, the acylation reagent used to obtain R₁ is selected from R₁-halogen, R₁-O-R₁ or 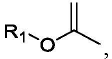 and the acylation reagent used to obtain R₂ is selected from R₂-halogen, R₂-O-R₂ or

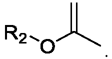

In another preferred embodiment, the acylation reagent used to obtain R₁ is selected from C₁-C₆ alkyl acyl chlorides, substituted or unsubstituted benzoic anhydrides, isopropenyl acetate, or trimethylchlorosilane.

In another preferred embodiment, the acylation reagent used to obtain R₂ is selected from isopropenyl acetate, or substituted or unsubstituted benzoic anhydride.

The present invention further provides a method for preparing abiraterone, wherein the method comprises the steps of: (2) deprotecting the compound of formula I prepared by the above preparation method to obtain abiraterone, and the reaction formula is as follows:

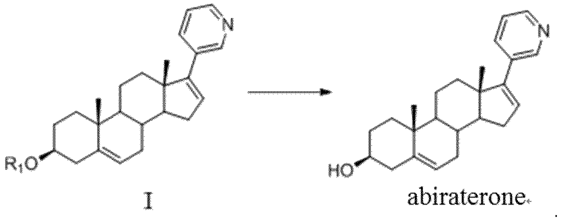

The present invention further provides a method for preparing abiraterone acetate, wherein the method comprises the steps of: (3) reacting the abiraterone prepared by the above preparation method with acetic anhydride to obtain abiraterone acetate, and the reaction formula is as follows:

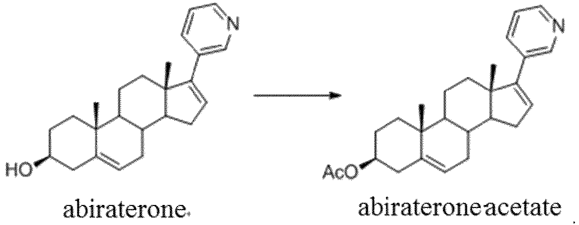

### DETAILED DESCRIPTION OF THE INVENTION

After the extensive and in-depth research, the inventor provided a method for preparing abiraterone acetate and an intermediate thereof through a large number of screenings and tests. Compared with the prior art, the method of the present invention is easy to operate, high in safety, low in cost, higher in yield, and suitable for industrial production.

### Term

Unless otherwise defined, all technical and scientific terms used herein have the same meanings as those commonly understood by those skilled in the art to which the present invention belongs.

As used herein, the terms "containing" or "including (comprising)" can be open, semi-closed, or closed. In other words, the term also includes "substaintially consisting of" or "consisting of".

As used herein, the term "room temperature" or "normal temperature" refers to a temperature of 4-40°C, preferably 25 ± 5°C.

Unless otherwise stated, the term "alkyl" itself or as a portion of another substituent refers to a straight or branched hydrocarbyl with a specified number of carbon atoms. Examples of alkyl include methyl, ethyl, n-propyl, isopropyl, n-butyl, tert-butyl, isobutyl, sec-butyl, n-pentyl, n-hexyl, n-heptyl, n-octyl, etc. C₁-C₆ alkyl represents an alkyl group containing 1~6 carbons, while C₁-C₃ alkyl represents an alkyl containing 1~3 carbons.

Unless otherwise stated, the term "acyl", used alone or as a portion of another group, refers to the group whose two hydrogens on the carbon at the connection point are substituted by a substituent of =O. The term "C₁-C₆ acyl" represents C₁-C₆ alkyl-(C=O)-.

Unless otherwise stated, the term "halogen" includes fluorine, chlorine, bromine, and iodine.

Unless otherwise specified, the term "solution" refers to an aqueous solution. Unless otherwise specified, solution refers to mass concentration.

Palladium salts that can be used in the present invention include but are not limited to bis triphenylphosphine palladium chloride, palladium acetate, palladium chloride, palladium trifluoromethanesulfonate, or combinations thereof. Copper salts that can be used in the present invention include but are not limited to cuprous iodide, copper acetate, copper chloride, or combinations thereof. Cobalt salts that can be used in the present invention include but are not limited to cobalt chloride, cobalt acetylacetonate, cobalt acetate, and cobalt sulfate. The nickel salts that can be used in the present invention include but are not limited to nickel chloride, nickel bromide, nickel acetate, and tricyclohexylphosphine nickel chloride. Iron salts that can be used in the present invention include but are not limited to ferric chloride, ferrous chloride, ferric acetylacetonate, and ferrous acetylacetonate.

The ligands that can be used in the present invention include but are not limited to phosphorus- containing ligands, amino acid ligands, and nitrogen-containing ligands. "Phosphorus-containing ligand" refers to a ligand containes phosphorus and can form a coordination catalyst with a metal catalyst to increase the activity of the catalyst. The phosphorus-containing ligand includes but is not limited to triphenylphosphine, tricyclohexylphosphine, tri tert-butylphosphine, and derivatives thereof. "Amino acid-containing ligand" refers to a ligand containes amino acids and can form a coordination catalyst with a metal catalyst to increase the activity of the catalyst. The amino acid containing ligand includes but is not limited to L-proline, alanine, methionine, and derivatives thereof. "Pyridine-containing ligand" refers to a ligand containes a pyridine and can form a coordination catalyst with a metal catalyst to increase the activity of the catalyst. The pyridine- containing ligand includes but is not limited to pyridine, 2,2-biphyridine, 1,10-phenanthroline, and derivatives thereof.

In the description of the present invention, "electrolyte" can be dissolved in the solvent used in the electrochemical reaction and conduct electricity. The electrolytes that can be used in the present invention include but are not limited to tetraethyl ammonium perchlorate, tetraethyl ammonium p-toluenesulfonate, tetrabutyl ammonium acetate, tetrabutyl ammonium hexafluorophosphate, tetrabutyl ammonium tetrafluoroborate, tetraethyl ammonium tetrafluoroborate, tetraethyl ammonium hexafluorophosphate, or combinations thereof.

The additives that can be used in the present invention include but are not limited to alkali metal salts. The alkali metal salts include but are not limited to potassium chloride, potassium carbonate, potassium tert-butoxide, potassium bicarbonate, potassium phosphate, potassium acetate, sodium carbonate, sodium bicarbonate, sodium acetate, lithium chloride, and lithium carbonate.

### Preparation method of compound of formula I

As the first aspect of the present invention, the method for preparing abiraterone acetate or an intermediate thereof comprises the steps of: in an organic solvent, in the presence of a metal catalyst, ligand, and reducing agent, reacting a 3-site protected 17-hydroxy ester androst-5,16-diene- 3β-hydroxy (ester) (compound of formula II) with 3-halogenated pyridine (compound of formula III) to obtain abiraterone acetate or abiraterone derivatives (compound of formula I), which can be converted to abiraterone acetate.

The reaction formula is as follows:

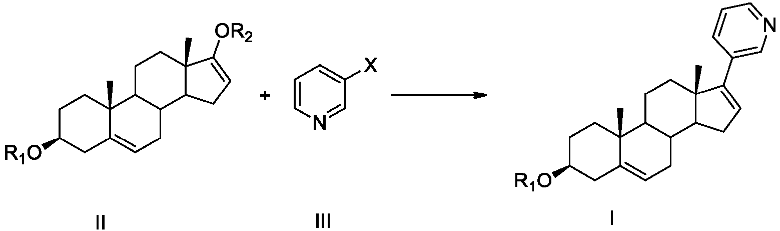

R₁ is selected from hydrogen or a protecting group consisting of C₁-C₆ alkyl, phenyl, benzyl, C₁-C₆ acyl, substituted or unsubstituted benzoyl, methylsulfonyl, substituted or unsubstituted benzenesulfonyl, and C₁-C₂ trialkylsilyl.

R₂ is a leaving group consisting of C₁-C₆ acyl, substituted or unsubstituted benzoyl, methylsulfonyl, substituted or unsubstituted benzenesulfonyl, dimethylamino acyl, diethylaminoacyl, or diphenylphosphinyl acyl.

X is fluorine, chlorine, bromine, or iodine.

The molar ratio of compound of formula II to compound of formula III is preferably 1: 1~4, more preferably 1: 1.5~3, such as 1: 2 or 1: 3.

The molar ratio of compound of formula II to metal catalyst is preferably 1: 0.005~0.3; more preferably 1: 0.01~0.2, such as 1: 0.03, 1: 0.05; 1: 0.07 or 0.1.

The molar ratio of compound of formula II to the ligand is preferably 1: 0.005~0.3; more preferably 1: 0.01~0.2, such as 1: 0.03, 1: 0.05; 1: 0.07 or 0.1.

The molar ratio of compound of formula II to the reducing agent is preferably 1: 1-4, more preferably 1: 1.5~3, such as 1: 2 or 1: 3.

The metal catalyst is selected from metal salt catalysts such as palladium, copper, iron, cobalt, nickel salt, etc., such as bis triphenylphosphine palladium chloride, palladium acetate, palladium chloride, cuprous iodide, copper acetate, ferric chloride, ferric acetylacetonate, cobalt chloride, acetylacetonate cobalt, nickel acetate, nickel chloride, tricyclohexylphosphine nickel chloride, etc.

The ligand is selected from phosphorus-containing ligands such as triphenylphosphine and tricyclohexylphosphine, amino-acid ligands such as L-proline, and nitrogen-containing ligands such as pyridine, 2,2-bipyridine, and 1,10-phenanthroline. The role of the ligand is to increase the activity of the catalyst.

The reducing agent is diboron reagent, as well as metal powders such as zinc powder, copper powder, iron powder, magnesium powder, manganese powder, tin powder, samarium powder, indium powder, etc.

The organic solvent is selected from tetrahydrofuran, toluene, acetonitrile, N,N-dimethylformamide, N,N-dimethylacetamide, N-methylpyrrolidone, dimethyl sulfoxide, or combinations thereof.

As the second aspect of the present invention, the method (electrochemical preparation method) for preparing abiraterone acetate or an intermediate thereof comprises the following steps: (1) in a solvent, in the presence of a metal catalyst, ligand, and electrolyte, under the constant current, reacting the compound of formula II with the compound of formula III to obtain the compound of formula I, and the reaction formula is as follows: 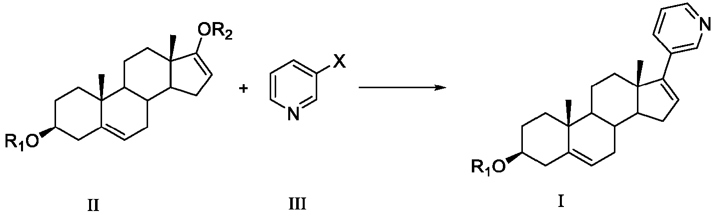
wherein, R₁ is selected from hydrogen, C₁-C₆ alkyl, phenyl, benzyl, C₁-C₆ acyl, substituted or unsubstituted benzoyl, methylsulfonyl, substituted or unsubstituted benzenesulfonyl, or C₁-C₂ trialkylsilyl or dialkylarylsilyl, and the alkyl in the dialkylarylsilyl is C₁-C₃ alkyl;
R₂ is selected from C₁-C₆ acyl, substituted or unsubstituted benzoyl, methylsulfonyl, substituted or unsubstituted benzenesulfonyl, dimethylamino acyl, diethylamino acyl, or diphenylphosphinic;
the term "substituted" refers to that one or more hydrogen atoms on a group are independently substituted by a group selected from the group consisting of:
   halogen, C₁-C₄ alkyl or C₁-C₄ haloalkyl, ether, nitro;
   X is fluorine, chlorine, bromine or iodine;
   the metal catalyst is selected from palladium salts, copper salts, cobalt salts, nickel salts, or combinations thereof; and
   the ligand is selected from phosphorus-containing ligands, amino acid-containing ligands, pyridine-containing ligands, or combinations thereof.

The molar ratio of the compound of formula II to the compound of formula III is preferably 1: 1~4, more preferably 1: 1~3, and most preferably 1: 1.5~2.2, such as 1: 1.8 or 1: 2.5;
The molar ratio of the compound of formula II to the metal catalyst is preferably 1: 0.005~0.2, more preferably 1: 0.01~0.1, and most preferably 1: 0.03~0.07, such as 1: 0.04, 1: 0.005, or 1: 0.006;
The molar ratio of the compound of formula II to the ligand is preferably 1: 0.005-0.3, more preferably 1: 0.01-0.2, and most preferably 1: 0.05-0.12, such as 1: 0.06, 1: 0.08, 1:0.10.

The molar volume of electrolyte to reaction solution is preferably 0.1~0.5 mol/L.

The constant current is preferably 0.05A~1.0A, and more preferably 0.1A~0.5A.

The current density is preferably 0.01~0.2A/cm², and more preferably 0.02~0.1A/cm².

The reaction temperature is preparely 0 °C~60 °C, and preparely 10 °C~50 °C.

The metal catalysts include but are not limited to bis triphenylphosphine palladium chloride, palladium acetate, palladium chloride, palladium trifluoromethanesulfonate, cuprous iodide, copper acetate, copper chloride, cobalt chloride, cobalt acetylacetonate, cobalt acetate, cobalt sulfate, nickel acetate, tricyclohexylphosphine nickel chloride, or combinations thereof.

The ligands include but are not limited to triphenylphosphine, tricyclohexylphosphine, tri tert-butylphosphine, L-proline, alanine, methionine, pyridine, 2,2-biphyridine, 1,10-phenanthroline, or combinations thereof.

The electrolytes include but are not limited to tetraethyl ammonium perchlorate, tetraethyl ammonium p-toluenesulfonate, tetrabutyl ammonium acetate, tetrabutyl ammonium hexafluorophosphate, tetrabutyl ammonium tetrafluoroborate, tetraethyl ammonium tetrafluoroborate, tetraethyl ammonium hexafluorophosphate, or combinations thereof.

The solvents used in the reaction include but are not limited to acetonitrile, N,N-dimethylformamide, N,N-dimethylacetamide, N-methylpyrrolidone, dimethyl sulfoxide, or combinations thereof.

The anode electrode includes but is not limited to iron, zinc, magnesium, nickel, aluminum or alloys thereof, and the cathode is platinum.

During the preparation process of the compound of formula I of the present invention, the reaction can be detected for completion by conventional methods in the field, such as detecting the disappearance of the main raw material (i.e., the compound of formula II) by TLC, which is the endpoint of the reaction.

As the third aspect of the present invention, the method for preparing abiraterone acetate or an intermediate thereof comprises the steps of: in a solvent, in the presence of a metal catalyst, ligand, and base, reacting a 3-site protected 17-hydroxy ester androst-5,16-diene-3β-hydroxy (ester) (compound of formula II) with 3-halogenated pyridine (compound of formula III-1) to obtain abiraterone acetate or an intermediate thereof (compound of formula I), and the intermediate can be converted to obtain abiraterone acetate.

The reaction formula is as follows: 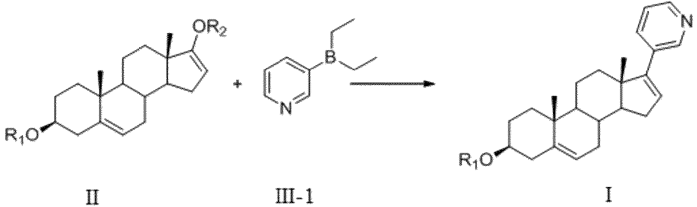 wherein, R₁ is selected from hydrogen, or a protecting group consisting of C₁-C₆ acyl, phenyl, benzyl, C₁-C₆ acyl, alkyl, substituted or unsubstituted benzoyl, trialkylsilyl, and dialkylarylsilyl, and the alkyl in trialkylsilyl and dialkylarylsilyl is C₁-C₃ alkyl. R₂ is selected from leaving groups such as C₁-C₆ acyl, substituted or unsubstituted benzoyl, dimethylamino acyl, diethylamino acyl, or diphenylphosphinyl acyl. R₁ and R₂ can be the same or different. The term "substituted" refers to that one or more hydrogen atoms on a group are independently substituted by a group selected from the group consisting of: halogen, C₁-C₄ alkyl, C₁-C₄ haloalkyl, ether, nitro, etc.

The molar ratio of the compound of formula II to the compound of formula III-1 is not particularly limited, and can be used according to the conventional dosage for such reactions, preferably 1: 1~3, more preferably 1: 1~1.5, such as 1: 1.02, 1: 1.05, 1: 1.1, 1: 2, 1: 3, 1: 4, 1: 5.

The molar ratio of the compound of formula II to the metal catalyst is not particularly limited, and can be used in the conventional dosage for such reactions, preferably 1: 0.005~0.3; more preferably 1: 0.01~0.2, such as 1: 0.03, 1: 0.05; 1: 0.07, 1: 0.1.

The molar ratio of the compound of formula II to the ligand is not particularly limited, and can be used in the conventional dosage for such reactions, preferably 1: 0.005~0.3; more preferably 1: 0.01~0.2, such as 1: 0.01, 1: 0.02, 1: 0.05, 1: 0.06, 1: 0.08, 0.1.

The molar ratio of the compound of formula II to the base is not particularly limited, and can be used in the conventional dosage for such reactions, preferably 1: 1-3; more preferably 1:1~2, such as 1: 1, 1: 1.5, or 1: 2.

The bases that can be used in the present invention include but are not limited to inorganic bases such as potassium carbonate, sodium carbonate, potassium phosphate, lithium tert-butoxide, sodium hydroxide, sodium bicarbonate, etc. The function of base is to act as an acid binding agent to absorb the acetate ions after the reaction, promoting the progress of the reaction.

The solvents that can be used in the present invention include but are not limited to tetrahydrofuran, toluene, acetonitrile, N,N-dimethylformamide, N,N-dimethylacetamide, N-methylpyrrolidone, and dimethyl sulfoxide.

As the fourth aspect of the present invention, the method for preparing abiraterone acetate or an intermediate thereof comprises the steps of: in a solvent, in the presence of a metal catalyst, reacting the compound of formula II with the compound of formula III-2 to obtain the compound of formula I.

The reaction formula is as follows:

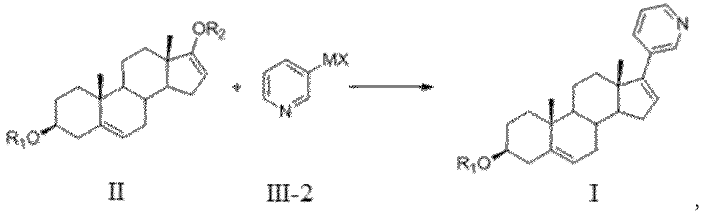

R₁ is selected from hydrogen, C₁-C₆ alkyl, phenyl, benzyl, C₁-C₆ acyl, substituted or unsubstituted benzoyl, methylsulfonyl, substituted or unsubstituted benzenesulfonyl, trialkylsilyl or dialkylarylsilyl, and the alkyl group in trialkylsilyl and dialkylarylsilyl is C₁-C₃ alkyl; R₂ is selected from C₁-C₆ acyl, substituted or unsubstituted benzoyl, methylsulfonyl, substituted or unsubstituted benzenesulfonyl, dimethylamino acyl, diethylaminoacyl, or diphenylphosphinyl acyl; M is a metal ion such as magnesium, iron, zinc, copper, tin, manganese, bismuth, or indium; R₁ and R₂ can be the same or different; X is fluorine, chlorine, bromine, iodine, or a coordinated anion of M, preferably, the coordinated anion of M is selected from pivalate or acetate, and the metal catalyst is selected from palladium salts, copper salts, iron salts, cobalt salts, nickel salts, or combinations thereof.

In a specific embodiment, the reaction system of the above preparation method further comprises ligands and/or additives. The ligand is selected from phosphorus-containing ligands, amino acid ligands, nitrogen-containing ligands, or combinations thereof, and the additive is selected from alkali metal salts. The role of ligands and additives is to increase the activity of the reaction, and the compound of formula II can also react with the compound of formula III-2 without the addition of ligands and additives.

The molar ratio of the compound of formula II to the compound of formula III-2 is not particularly limited, and can be used according to the conventional dosage for such reactions, preferably 1: 1~4, more preferably 1: 1~2, such as 1: 1.2, 1: 1.5, 1: 1.8, 1: 2.0. The molar ratio of the compound of formula II to the metal catalyst is not particularly limited, and can be used according to the conventional dosage for such reactions, preferably 1: 0.005-0.3, more preferably 1: 0.01-0.2, such as 1: 0.03, 1: 0.05; 1: 0.07, 1: 0.1. The molar ratio of the compound of formula II to the ligand is not particularly limited and can be used in the conventional dosage for such reactions, preferably 1: 0.005~0.3; more preferably 1: 0.01~0.2, such as 1: 0.01, 1: 0.02, 1: 0.05, 1: 0.06, 1: 0.08, 0.1. The molar ratio of the compound of formula II to the additive is not particularly limited, and can be used according to the conventional dosage for such reactions, preferably 1: 0.1~3, and more preferably 1: 1~2, such as 1: 1.2, 1: 1.5, 1: 1.8, and 1: 2.0.

The temperature for dropwise addition of the compound of III-2 is -30 °C~25 °C, the reaction temperature is 0 °C~70 °C, preferably, the reaction temperature is room temperature ~50 °C.

The solvents used in the reaction include but are not limited to tetrahydrofuran, 2-methyltetrahydrofuran, ethylene glycol dimethyl ether, methyl tert-butyl ether, ether, and toluene.

The "metal catalysts" that can be used for this preparation method are metal salts, including but not limited to palladium salts, copper salts, iron salts, cobalt salts, and nickel salts.

The "ligands" that can be used for this preparation method include but are not limited to phosphorus-containing ligands, amino acid ligands, and nitrogen-containing ligands.

The "additives" that can be used for this preparation method include but are not limited to alkali metal salts.

### Preparation method of compound of formula II

Typically, the compound of formula II can be obtained by an acylation at positions 3 and 17 in dehydroepiandrosterone. There are no specific requirements for the acylation method in the present invention, and commonly used methods in the field or methods referring to the present invention can be used.

Preferably, the method further comprises the steps of:
(i-a) reacting the compound of formula IV with an acylation reagent to obtain the compound of formula II;
the reaction formula is as follows: 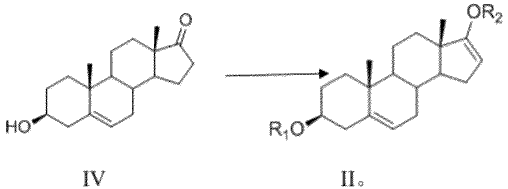
wherein, the acylation reagent used to obtain R₁ is selected from the group consisting of: R₁-halogen, R₁-O-R₁, or combinations thereof. The acylation reagent used to obtain R₂ is selected from the group consisting of: R₂-halogen, R₂-O-R₂, or combinations thereof.

When R₁ is acetyl, the compound of formula I is abiraterone acetate.

When R₁ is not acetyl, the compound of formula I is a derivative of abiraterone.

Preferably, abiraterone derivatives can be converted to abiraterone acetate through the following steps:
(ii) in a alcohol solvent, an abiraterone derivative undergoes a hydrolysis reaction with sodium hydroxide solution to obtain abiraterone; and
(iii) abiraterone undergoes an esterification reaction with acetic anhydride to obtain abiraterone acetate.

In step (ii), the alcohol solvent is selected from methanol, ethanol, isopropanol, or combinations thereof.

### Preparation method of compound of formula III-2

Typically, the compound of formula III-2 can undergo Grignard exchange with 3-bromopyridine and isopropylmagnesium chloride, followed by the addition of a metal salt or metal powder for metalization to obtain pyridine metal salt. There are no specific requirements for the preparation method of the compound of formula III-2 in the present invention, and commonly used methods in the technical field or methods referring to the present invention can be used. In a specific embodiment of the present invention, the reaction formula for preparing the compound of formula III-2 is as follows: 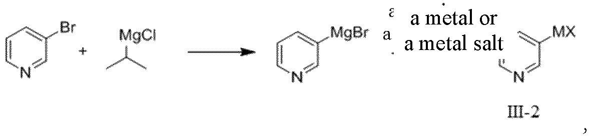 wherein, 3-bromopyridine can be replaced by low-cost 3-chloropyridine, and generally, the solvent is a ether solvent such as tetrahydrofuran. The successfully prepared pyridine metal salt solution should be used as soon as possible to avoid deterioration.

### Preparation method of compound of formula II

Typically, the compound of formula II can be obtained by an acylation at positions 3 and 17 in dehydroepiandrosterone. There are no specific requirements for the acylation method in the present invention, and commonly used methods in the field or methods referring to the present invention can be used.

Preferably, the method further comprises the steps of:
(i-a) reacting the compound of formula IV with an acylation reagent to obtain the compound of formula II;
the reaction formula is as follows: 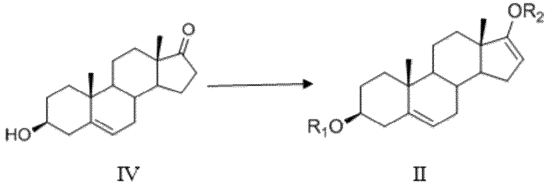
wherein, the acylation reagent used to obtain R₁ is selected from the group consisting of: R₁-halogen, R₁-O-R₁, or 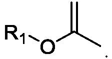 The acylation reagent used to obtain R₂ is selected from the group consisting of: R₂-halogen, R₂-O-R₂, or

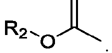

When R₁ is acetyl, the compound of formula I is abiraterone acetate.

When R₁ is not acetyl, the compound of formula I is an intermediate or a derivative of abiraterone.

In the preparation process of the compound of formula II of the present invention, the completion of the reaction can be detected by conventional methods in the field, such as TLC method, with the disappearance of the main raw material (i.e., the compound of formula IV) as the endpoint of the reaction.

### The main advantages of the present invention include:

The method of the present invention overcomes the drawbacks of expensive raw materials, high costs, and harsh reaction conditions in the prior art, and the method of the present invention is low in cost, high in yield, simple in production method, and suitable for industrial production of abiraterone acetate, and has great application value.

The present invention will be explained in more detail below in combined with examples. The examples of the present invention are only used to illustrate the technical solution of the present invention, and the essence and scope of the present invention are not limited to them. Unless otherwise specified, percentages and parts are weight percentages and weight parts.

### Example 1

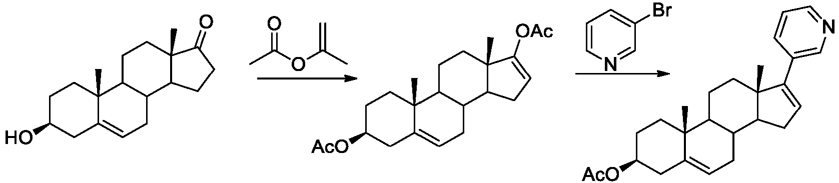

1.44kg (5.0mol) of dehydroepiandrosterone, 86.0g (0.5mol, 0.1equiv) of p-toluenesulfonic acid, and 10.0 L of isopropenyl acetate were added to a reaction flask, and the reaction was heated up to 120 ± 5 °C and distilled at atmospheric pressure for 2 hours. Until the volume of the remaining material was about 3L, the distillation stopped, and it continued to be stirred for 3 hours at this temperature, cooled down to 0~5 °C, stirred for 30 minutes, filtered, and 5L of water was added to the filter cake, and it was stirred for 30 minutes, filtered, and 3L of ethanol was added to the filter cake, and it was pulped at room temperature for 30 minutes, cooled down to 0~5 °C, stirred for 30 minutes, filtered, and the filter cake was rinsed with a small amount of cold ethanol, dried under vacuum at 35-40 °C to obtain 1.72Kg of 17-acetate androst-5,16-diene-3β-acetate, with a yield of 92.2% by molar and a purity of 99.2% by HPLC.

744.0g (2.0 mol) of 17-acetate androst-5,16-diene-3β-acetate, 474.0g (3.0 mol) of 3-bromopyridine, 14.1g (0.02 mol) of PdCl₂(PPh₃)₂, 15.6g (0.10 mol) of 2,2-bipyridine, 508.0g (2.0 mol) of bis (pinacol) diborone, and 5.0L of DMF were added to a reaction flask. After nitrogen replacement, the reaction was heated up to 60 °C and stirred for 5 hours. The reaction was monitored by TLC, and after it was completed, the reaction mixture was cooled down to room temperature, transferred slowly to a 50L reactor containing 20L of drinking water, stirred for 30 minutes, filtered, and 10L of dichloromethane was added to the filter cake to allow it dissolved, and then it was washed sequentially with 2L of 1N hydrochloric acid aqueous solution, 2L of 1N sodium bicarbonate aqueous solution and 2L of water. The organic layer was concentrated to dryness under reduced pressure, and 3L of 90% ethanol aqueous solution was added to the concentrate. It was heated to reflux to allow it dissoved and clear, cooled down to 0~5 °C within two hours, crystallized, filtrated, and dried under vacuum to obtain 731.2g of crude abiraterone acetate, with a yield of 93.5% by molar and a purity of 98.7% by HPLC. The crude product was recrystallized from acetone to obtain 685.9g of abiraterone acetate, with a purity of 99.8%.

ESI-MS(m/z):414 [M+Na]⁺; 1H NMR (400MHz, DMSO-d6) δ(ppm): 8.933(d, 1H), 8.822(d, 1H), 8.648(d, 1H), 8.072(dd, 1H), 6.568(s, 1H), 5.399(s, 1H), 4.485 (m, 1H), 2.322 (m, 3H), 2.146 (m, 2H), 2.049 (m, 1H), 1.994(s, 3H), 1.863 (m, 2H), 1.741 (m, 3H), 1.601 (m, 3H), 1.450 (m, 1H), 1.114 (m, 2H), 1.055 (s, 3H), 1.046(s, 3H).

### Example 2

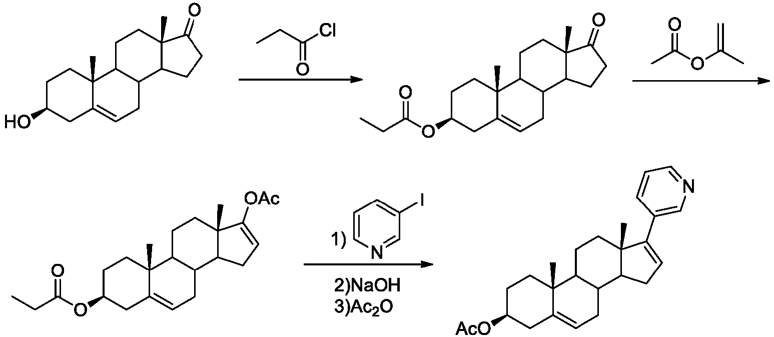

14.4g (0.05mol) of dehydroepiandrosterone, 100mL of dichloromethane, and 6.1g (0.06mol) of triethylamine were added to a reaction flask, and the mixture was stirred until dissolved and clear, cooled down to 0~5 °C, and 4.9g (52.5mmol) of propionyl chloride was added dropwise and slowly. After the addition was completed, it was heated up to room temperature and stirred at this temperature for 3 hours. The reaction was monitored by TLC, and after it was completed, 50mL of drinking water was added slowly. The water layer was removed, and the organic layer was dried with anhydrous sodium sulfate, filtered, and the filtrate was concentrated to dryness. 0.9g (5.0mmol) of p-toluenesulfonic acid and 100mL of isopropenyl acetate were added to the concentrate, and the reaction solution was heated to 120 ± 5 °C and distilled at atmospheric pressure for 1 hour. Until the volume of the remaining material was about 50mL, the distillation stopped, and it continued to be stirred for 2 hours at this temperature, cooled down to 0~5 °C, stirred for 30 minutes, filtered, and 80mL of water was added to the filter cake, and it was stirred for 30 minutes, filtered, and 30mL of ethanol was added to the filter cake, and it was pulped at room temperature for 30 minutes, cooled down to 0~5 °C, stirred for 30 minutes, filtered, and the filter cake was rinsed with a small amount of cold ethanol, dried under vacuum at 35~40 °C to obtain 17.7g of 17-acetate androst-5,16-diene-3 β-propionate, with a yield of 91.5% by molar and a purity of 99.3% by HPLC.

7.7g (0.02 mol) of 17-acetate androst-5,16-diene-3β-propionate, 4.1g (0.02 mol) of 3-iodopyridine, 0.2g (1.0mmol) of nickel acetate, 0.3g (1.0mmol) of tricyclohexylphosphine, 2.6g (0.04 mol) of zinc powder, and 50mL of acetonitrile were added to a reaction flask. After nitrogen replacement, it was stirred vigorously, heated to reflux, and refluxed for 5 hours. The reaction was monitored by TLC, and after it was completed, it was filtered while hot, and the filtrate was concentrated to dryness. 50mL of ethanol and 10mL of 30% sodium hydroxide solution was added to the concentrate, and it was heated to reflux, refluxed for 3 hours, cooled down to room temperature, and 100mL of drinking water was added to age for 30 minutes. It was filtrated, and dried under vacuum to abtain 6.4 g of abiraterone, with a yield of 91.5% by molar. ESI-MS(m/z):372[M+Na]⁺; ¹H NMR (400MHz, DMSO-d6) δ(ppm): 8.584(s,1H), 8.434(d,1H), 7.762(d,1H), 7.342(q,1H), 6.109 (s,1H), 5.301 (s, 1H), 4.628 (d,1H), 2.190 (m,3H), 2.066 (m,3H), 1.771 (m,1H), 1.696 (m,4H), 1.534(m,2H), 1.372 (m,2H), 1.012 (m,2H), 0.983 (s,3H), 0.965(s,3H);
6.4g (0.018mol) of abiraterone, 50mL of dichloromethane, and 3.0g (0.03mol) of triethylamine was added to a reaction flask. The reaction was cooled down to 0~5 °C and 3.0g (0.03mol) of acetic anhydride was added dropwise and slowly. After the addition was completed, it was heated up to room temperature, reacted at this temperature for 3 hours, filtered, and the organic layer was washed once with 20 mL of water, and 20 mL of 1N sodium bicarbonate aqueous solution. The organic layer was concentrated to dryness under reduced pressure, and 20mL of 90% ethanol aqueous solution was added to the concentrate, and it was heated to reflux to allow it dissoved and clear, cooled down to 0~5 °C within two hours, crystallized, filtrated, and dried under vacuum to obtain 6.8g of crude abiraterone acetate, with a yield of 96.7% by molar and a purity of 98.3% by HPLC. The crude product was recrystallized from acetone to obtain 6.2g of abiraterone acetate, with a purity of 99.6%.

ESI-MS(m/z):414 [M+Na]⁺; ¹H NMR (400MHz, DMSO-d6) δ(ppm): 8.935(d,1H), 8.825(d,1H), 8.650(d,1H), 8.075(dd,1H) , 6.571(s,1H), 5.402(s, 1H), 4.487 (m,1H), 2.324 (m,3H), 2.148(m,3H), 1.996(s,3H), 1.865 (m,2H), 1.743 (m,3H), 1.602 (m,4H), 1.116 (m,2H), 1.058 (s,3H), 1.046(s,3H).

### Example 3

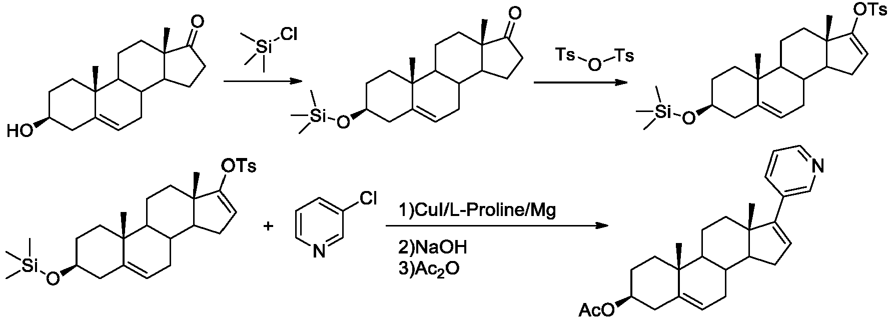

14.4g (0.05mol) of dehydroepiandrosterone, 100mL of dichloromethane, and 6.1g (0.06mol) of triethylamine were added to a reaction flask, and the mixture was stirred until dissolved and clear. The reaction was cooled down to 0~5 °C and 5.7g (52.5mmol) of trimethylchlorosilane was added dropwise and slowly. After the addition was completed, it was heated up to room temperature and stirred at this temperature for 4 hours. The reaction was monitored by TLC, and after it was completed, 50mL of drinking water was added slowly. The water layer was removed, and the organic layer was dried with anhydrous sodium sulfate, filtered, and the filtrate was concentrated to dryness. 5.6g (0.05mol) of potassium tert-butoxide and 100mL of tetrahydrofuran was added to the concentrate, and it was stirred for 10 minutes, cooled down to 0~5 °C, and 16.3g (0.05mol) of p-toluenesulfonic anhydride was added slowly, and it was heated up to 60~65 °C, and stirred at this temperature for 5 hours. The reaction was monitored by TLC, and after it was completed, it was concentrated under reduced pressure until the volume of the remaining material was about 50mL, and 80mL of water was added, and it was stirred for 30 minutes, filtered, and 30mL of ethanol was added to the filter cake, and it was pulped at room temperature for 30 minutes, cooled down to 0~5 °C, stirred for 30 minutes, filtered, and the filter cake was rinsed with a small amount of cold ethanol, and dried under vacuum at 35~40 °C to obtain 21.2g of 17-p-tosylate androst-5,16-diene-3β-trimethylsilyl ester, with a yield of 82.6% by molar and a purity of 98.3% by HPLC.

10.3g (0.02 mol) of 17-p-tosylate androst-5,16-diene-3β-trimethylsilyl ester, 6.8g (0.06 mol) of 3-chloropyridine, 0.8g (4.0mmol) of cuprous iodide, 0.8g (4.0mmol) of L-proline, 1.5g (0.06 mol) of magnesium powder, and 50mL of DMSO were added to a reaction flask. After nitrogen replacement, it was stirred vigorously, heated up to 40 °C, and stirred for 3 hours. The reaction was monitored by TLC, and after it was completed, the reaction mixture was cooled down to room temperature, dripped slowly into 200mL of drinking water, stirred for 30 minutes, filtered, and 50mL of ethanol and 10mL of 30% sodium hydroxide solution were added to the filter cake, it was heated up to reflux, stirred for 5 hours, cooled down to room temperature, and 100mL of drinking water was added to age for 30 minutes. It was filtrated and dried under vacuum to abtain 5.8 g of abiraterone, with a yield of 82.0% by molar.

5.8g (0.016mol) of abiraterone, 50mL of dichloromethane, and 3.0g (0.03mol) of triethylamine were added to a reaction flask. The reaction was cooled down to 0~5 °C, and 3.0g (0.03mol) of acetic anhydride was added dropwise and slowly. After the addition was completed, it was heated up to room temperature, reacted at this temperature for 3 hours, and filtered. The organic layer was washed once with 20 mL of water, and 20 mL of 1N sodium bicarbonate aqueous solution, concentrated to dryness under reduced pressure, and 20mL of 90% ethanol water was added to the concentrate. It was heated to reflux to allow it dissoved and clear, cooled down to 0~5 °C within two hours, crystallized, filtrated, and dried under vacuum to obtain 6.2g of crude abiraterone acetate, with a yield of 95.1% by molar and a purity of 98.0% by HPLC. The crude product was recrystallized from acetone to obtain 5.5g of abiraterone acetate, with a purity of 99.5%. ESI-MS (m/z): 414 [M+Na]⁺.

### Example 4

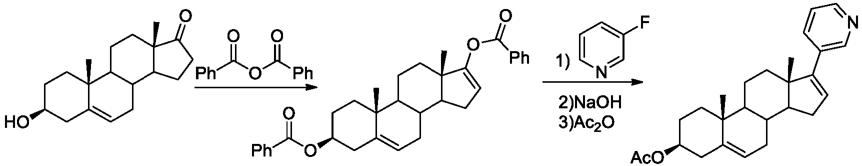

14.4g (0.05mol) of dehydroepiandrosterone, 11.2g (0.1mol) of potassium tert-butoxide, and 100mL of tetrahydrofuran were added to a reaction flask, and the mixture was stirred for 10 minutes, cooled down to 0~5 °C, and 22.6g (0.1mol) of benzoic anhydride was added slowly. It was heated up to 60~65 °C, and stirred at this temperature for 5 hours. The reaction was monitored by TLC, and after it was completed, it was concentrated under reduced pressure until the volume of the remaining material was about 50mL, and 80mL of water was added, and it was stirred for 30 minutes, filtered, and 50mL of water was added to the filter cake, and it was stirred for 30 minutes, filtered, and 30mL of ethanol was added to the filter cake, and it was pulped at room temperature for 30 minutes, cooled down to 0~5 °C and stirred for 30 minutes, filtered, and the filter cake was rinsed with a small amount of cold ethanol, and dried under vacuum at 35~40 °C to obtain 22.6g of 17-benzoate androst-5,16-diene-3β-benzoate, with a yield of 91.2% by molar and a purity of 98.6% by HPLC.

9.9g (0.02 mol) of 17-benzoate androst-5,16-diene-3β-benzoate, 3.9g (0.04 mol) of 3-fluoropyridine, 0.5g (4.0 mmol) of nickel chloride, 0.7g (4.0 mmol) of 1,10-phenanthroline, 3.3g (0.06 mol) of manganese powder, and 80mL of tetrahydrofuran were added to a reaction flask. After nitrogen replacement, it was stirred vigorously, heated up to 95~100 °C, and stirred for 6 hours. The reaction was monitored by TLC, and after it was completed, it was filtered while hot, and the filtrate was concentrated to dryness. 50mL of ethanol and 10mL of 30% sodium hydroxide solution were added to the concentrate, and it was heated to reflux, stirred for 3 hours, cooled down to room temperature, and 100mL of drinking water was added to age for 30 minutes. It was filtrated, and dried under vacuum to abtain 5.5 g of abiraterone, with a yield of 78.6% by molar.

5.5g (0.016mol) of abiraterone, 50mL of dichloromethane, and 3.0g (0.03mol) of triethylamine were added to a reaction flask. The reaction was cooled down to 0~5 °C, and 3.0g (0.03mol) of acetic anhydride was added dropwise and slowly. After the addition was completed, it was heated up to room temperature, reacted at this temperature for 3 hours, and filtered. The organic layer was washed once with 20 mL of water and 20 mL of 1N sodium bicarbonate aqueous solution, concentrated to dryness under reduced pressure, and 20mL of 90% ethanol water was added to the concentrate, and it was heated to reflux to allow it dissoved and clear, cooled down to 0~5 °C within two hours, crystallized, filtrated, and dried under vacuum to obtain 5.9g of crude abiraterone acetate, with a yield of 95.0% by molar and a purity of 98.8% by HPLC. The crude product was recrystallized from acetone solvent to obtain 5.4g of abiraterone acetate, with a purity of 99.8%.

ESI-MS (m/z): 414 [M+Na]⁺.

### Example 5

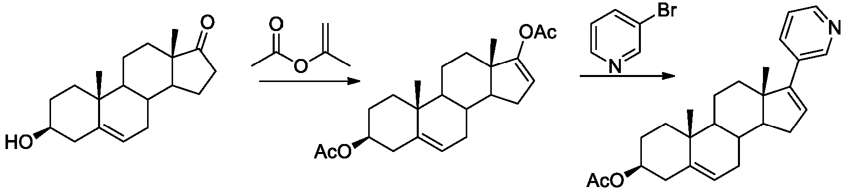

1.44kg (5.0mol) of dehydroepiandrosterone, 86.0g (0.5mol, 0.1equiv) of p-toluenesulfonic acid, and 10.0 L of isopropenyl acetate were added to a reaction flask, and the reaction was heated up to 120 ± 5 °C, and distilled at atmospheric pressure for 2 hours. Until the volume of the remaining material was about 3L, the distillation stopped, and it continued to be stirred for 3 hours at this temperature, cooled down to 0~5 °C, stirred for 30 minutes, filtered, and 5L of water was added to the filter cake, and it was stirred for 30 minutes, filtered, and the filter cake was added to 3L of ethanol, and it was pulped at room temperature for 30 minutes, cooled down to 0~5 °C, stirred for 30 minutes, filtered, and the filter cake was rinsed with a small amount of cold ethanol (200mL, 0~5 °C), and dried under vacuum at 35~40 °C to obtain 1.72Kg of 17-acetate androst-5,16-diene-3β-acetate, with a yield of 92.2% by molar and a purity of 99.2% by HPLC.

744.0g (2.0 mol) of 17-acetate androst-5,16-diene-3β-acetate, 474.0g (3.0 mol) of 3-bromopyridine, 14.1g (0.02 mol) of PdCl₂(PPh₃)₂, 31.2g (0.20 mol) of bipyridine, 5.0L of DMF, and 658.5g (2.0 mol) of tetrabutyl ammonium tetrafluoroborate were added to a reaction flask. The electrode used for electrochemistry (iron anode and platinum cathode) was fixed in the reaction flask, and the mixture was stirred at this temperature for 3 hours under constant current of 1.0A (current density of about 0.1A/cm²), and the reaction was completed. The reaction mixture was transferred lowly to a 50L reactor containing 20L of water, stirred for 30 minutes, filtered, and 10L of dichloromethane was added to the filter cake to allow it dissolved. It was washed sequentially with 2L of 1N hydrochloric acid aqueous solution, 2L of 1N sodium bicarbonate aqueous solution, and 2L of water. The organic layer was concentrated to dryness under reduced pressure, and 3L of ethanol was added to the concentrate. The mixture was heated to reflux to allow it dissoved and clear, cooled down to 0~5 °C at a constant speed within two hours, crystallized, filtrated, and dried under vacuum to obtain 678.8g of crude abiraterone acetate, with a yield of 86.8% by molar and a purity of 98.4% by HPLC. The crude product was recrystallized from acetone solvent to obtain 645.5g of abiraterone acetate, with a purity of 99.7%.

mp: 144.8 °C -146.3 °C (literature data, mp: 144°C-146°C). ESI-MS(m/z): 414 [M+Na]⁺; 1H NMR (400MHz, CDCl₃-d1) δ (ppm): ¹H NMR (500 MHz, CDCl₃) δ (ppm): 8.92 (s, 1H), 8.82 (d, 1H), 8.64 (d, 1H), 8.08~8.05 (dd, 1H), 6.56 (s, 1H), 5.40 (d, 1H), 4.48~4.42 (m, 1H), 2.31~2.27 (m, 3H), 2.15~2.09 (m, 2H), 2.04~2.0 (m, 1H), 1.99 (s, 3H), 1.86~1.75 (m, 2H), 1.74~1.66 (m, 3H), 1.66 1-1.52 (m, 3H), 1.44-1.39 (m, 1H), 1.11~1.01 (m, 2H), 1.05 (s, 3H), 1.04 (s, 3H). ^{13C} NMR (400MHz, CDCl₃-d1) δ (ppm): 170.58, 151.80, 148.01, 148.0, 140.14, 133.03, 133.75, 129.28, 123.09, 122.38, 73.94, 57.58, 50.37, 47.44, 36.89, 38.24, 37.02, 35.32, 31.89, 31.64, 30.52, 27.88, 21.61, 20.97, 19.35, 16.67.

### Example 6

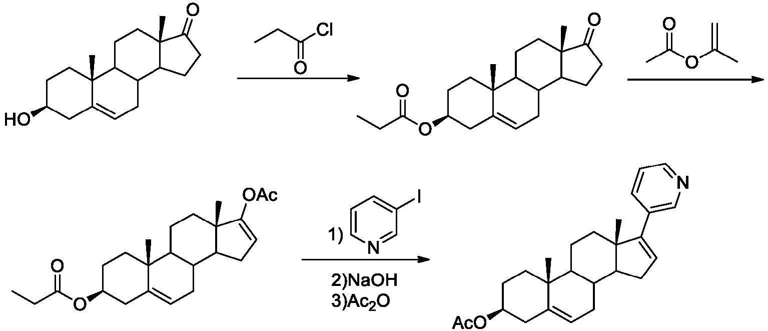

14.4g (0.05mol) of dehydroepiandrosterone, 100mL of dichloromethane, and 6.1g (0.06mol) of triethylamine were added to a reaction flask, and the mixture was stirred until dissolved and clear. It was cooled down to 0~5 °C, and 4.9g (52.5mmol) of propionyl chloride was added dropwise and slowly. After the addition was completed, it was heated up to room temperature and stirred at this temperature for 3 hours. The reaction was monitored by TLC, and after it was completed, 50mL of water was added to the reaction solution. The water layer was removed, and the organic layer was dried with anhydrous sodium sulfate, filtered, and the filtrate was concentrated to dryness. 0.9g (5.0mmol) of p-toluenesulfonic acid and 100mL of isopropenyl acetate were added to the concentrate, and the mixture was heated up to 120 ± 5 °C, and distilled at atmospheric pressure for 1 hour. Until the volume of the remaining material was about 50mL, the distillation stopped, and the it continued to be stirred for 2 hours at this temperature, cooled down to 0~5 °C, stirred for 30 minutes, filtered, and 80mL of water was added to the filter cake, and it was stirred for 30 minutes, filtered, and the filter cake was added to 30mL of ethanol, and it was pulped at room temperature for 30 minutes, cooled down to 0~5 °C, stirred for 30 minutes, filtered, and the filter cake was rinsed with a small amount of cold ethanol (5mL, 0~5 °C), and dried under vacuum at 35-40 °C to obtain 17.7g of 17-acetate androst-5,16-diene-3β-propionate, with a yield of 91.5% by molar and a purity of 99.3% by HPLC.

7.7g (0.02 mol) of 17-acetate androst-5,16-diene-3β-propionate, 4.1g (0.02 mol) of 3-iodopyridine, 0.2g (1.0mmol) of nickel acetate, 0.3g (1.0mmol) of tricyclohexylphosphine, and 50mL of acetonitrile were added to a reaction flask; and 651.0mg (3.0 mmol) of tetraethyl ammonium tetrafluoroborate was added. The electrodes used for electrochemistry (zinc anode and platinum cathode) was fixed in the reaction flask, and the reaction was stirred at 0-10 °C for 5 hours under a constant current of 0.05A (current density of about 0.01A/cm²), the reaction was completed, the mixture was filtered, and the filtrate was concentrated to dryness. 50mL of ethanol and 10mL of 30% sodium hydroxide solution were added to the concentrate, and it was heated to reflux, stirred for 3 hours, cooled down to room temperature, and 100mL of water was added to age for 30 minutes. It was filtrated and dried under vacuum to abtain 5.87 g of abiraterone, with a yield of 84.0% by molar.

5.87g (0.017mol) of abiraterone, 50mL of dichloromethane, and 3.0g (0.03mol) of triethylamine were added to a reaction flask. The reaction was cooled down to 0~5 °C and 3.0g (0.03mol) of acetic anhydride was added dropwise and slowly. After the addition was completed, it was heated up to room temperature, reacted at this temperature for 3 hours, and the abiraterone was completely reacted. The mixture was filtered, and the organic layer was washed sequentially with 20 mL of water and 20 mL of 1N sodium bicarbonate aqueous solution, concentrated to dryness under reduced pressure, and 20mL of 90% ethanol aqueous solution was added to the concentrate. The mixture was heated to reflux to allow it dissolved and clear, cooled down to 0~5 °C at a constant speed within two hours, crystallized, filtraed and dried under vacuum to obtain 6.2g of crude abiraterone acetate, with a yield of 96.5% by molar and a purity of 98.4% by HPLC. The crude product was recrystallized from acetone solvent to obtain 5.7g of abiraterone acetate, with a purity of 99.5%. ESI-MS (m/z): 414 [M+Na]⁺.

### Example 7

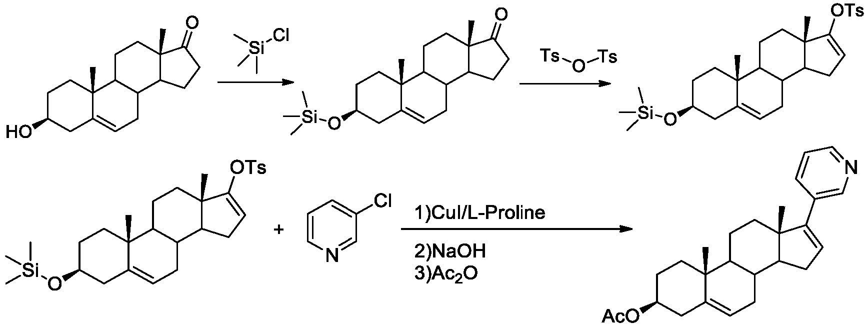

14.4g (0.05mol) of dehydroepiandrosterone, 100mL of dichloromethane, and 6.1g (0.06mol) of triethylamine were added to a reaction flask, and the mixture was stirred until it was dissolved and clear. It was cooled down to 0~5 °C and 5.7g (52.5mmol) of trimethylchlorosilane was added dropwise and slowly. After the addition was completed, it was heated up to room temperature and stirred at this temperature for 4 hours. The reaction was monitored by TLC, and after it was completed, 50mL of water was added slowly. The water layer was removed, and the organic layer was dired with anhydrous sodium sulfate, filtered, and the filtrate was concentrated to dryness. 5.6g (0.05mol) of potassium tert-butoxide and 100mL of tetrahydrofuran were added to the concentrate, and it was stirred for 10 minutes, cooled down to 0~5 °C, and 16.3g (0.05mol) of p-toluenesulfonic anhydride was added slowly, heated up to 60-65 °C, and stirred at this temperature for 5 hours. The reaction was monitored by TLC, and after it was completed, it was concentrated under reduced pressure until the volume of the remaining material was about 50mL, and 80mL of water was added, and it was stirred for 30 minutes, filtered, and the filter cake was added to 30mL of ethanol, and it was pulped at room temperature for 30 minutes, cooled down to 0~5 °C, stirred for 30 minutes, filtered, and the filter cake was rinsed with a small amount of cold ethanol (5mL, 0~5 °C), and dried under vacuum at 35-40 °C to obtain 21.2g of 17-p-tosylate androst-5,16-diene-3β-trimethylsilyl ester, with a yield of 82.6% by molar and a purity of 98.3% by HPLC.

10.3g (0.02 mol) of 17-p-tosylate androst-5,16-diene-3β-trimethylsilyl ester, 6.8g (0.06 mol) of 3-chloropyridine, 0.8g (4.0mmol) of cuprous iodide, 0.8g (4.0mmol) of L-proline, 50mL of DMSO, and 7.7g (2.0 mmol) of tetrabutyl ammonium hexafluorophosphate were added to a reaction flask. The electrodes used for electrochemistry (magnesium anode and platinum cathode) was fixed in the reaction flask, and it was stirred at 50 degrees for 5 hours under a constant current of 0.5A (current density of about 0.1A/cm²). The reaction was monitored by TLC, and after it was completed, the reaction mixture was cooled down to room temperature, added to 200mL of water dropwise and slowly, stirred for 30 minutes, filtered, and 50mL of ethanol and 10mL of 30% sodium hydroxide solution were added to the filter cake, and it was heated to reflux, stirred for 5 hours, cooled to room temperature, and 100mL of water was added to age for 30 minutes. It was filtrated and dried under vacuum to abtain 6.2 g of abiraterone, with a yield of 86.2% by molar.

6.2g (0.017mol) of abiraterone, 50mL of dichloromethane, and 3.0g (0.03mol) of triethylamine were added to a reaction flask, and the mixture was cooled down to 0~5 °C, and 3.0g (0.03mol) of acetic anhydride was added dropwise and slowly. After the addition was completed, it was heated up to room temperature, reacted at this temperature for 3 hours, and the abiraterone was completely reacted. The mixture was filtered, and the organic layer was washed sequentially with 20 mL of water and 20 mL of 1N sodium bicarbonate aqueous solution, concentrated to dryness under reduced pressure, and 20mL of 90% ethanol water was added to the concentrate. The mixture was heated to reflux to allow it dissolved and clear, cooled down to 0~5 °C at a constant speed within two hours, crystallized, filtrated, and dried under vacuum to obtain 6.4g of crude abiraterone acetate, with a yield of 95.1% by molar and a purity of 98.0% by HPLC. The crude product was recrystallized from acetone solvent to obtain 5.7g of abiraterone acetate, with a purity of 99.5%. ESI-MS (m/z): 414 [M+Na]⁺.

### Example 8

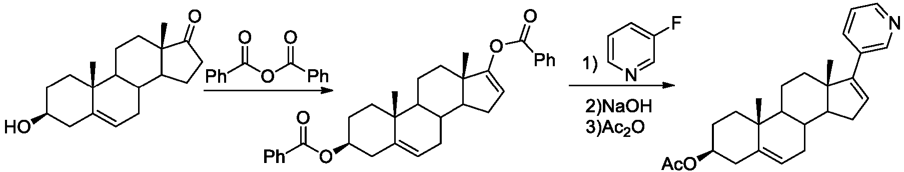

14.4g (0.05mol) of dehydroepiandrosterone, 11.2g (0.1mol) of potassium tert-butoxide, and 100mL of tetrahydrofuran were added to a reaction flask, and the mixture was stirred for 10 minutes, cooled down to 0~5 °C, and 22.6g (0.1mol) of benzoic anhydride was added slowly. It was heated up to 60-65 °C, and stirred at this temperature for 3 hours. The reaction was monitored by TLC, and after it was completed, the mixture was concentrated under reduced pressure until the volume of the remaining material was about 50mL, and 80mL of water was added, and it was stirred for 30 minutes, filtered, and 50mL of water was added to the filter cake, and it was stirred for 30 minutes, filtered, and the filter cake was added to 30mL of ethanol, and it was pulped at room temperature for 30 minutes, cooled down to 0~5 °C, stirred for 30 minutes, filtered, and the filter cake was rinsed with a small amount of cold ethanol (5mL, 0~5 °C), and dried under vacuum at 35-40 °C to obtain 22.6g of 17-benzoate androst-5,16-diene-3β-benzoate, with a yield of 91.2% by molar and a purity of 98.6% by HPLC.

9.9g (0.02 mol) of 17-benzoate androst-5,16-diene-3β-benzoate, 3.9g (0.04 mol) of 3-fluoropyridine, 0.5g (4.0 mmol) of nickel chloride, 0.7g (4.0 mmol) of 1,10-phenanthroline, 80mL of N, N-dimethylacetamide (DMAc), and 9.2g (0.04 mol) of tetraethyl ammonium perchlorate were added to a reaction flask. The electrodes used for electrochemistry (magnesium anode and platinum cathode) was fixed in the reaction flask, and the mixture was stirred at room temperature for 6 hours under a constant current of 0.3A (current density of about 0.06A/cm²). The reaction was monitored by TLC, and after it was completed, it was filtered, and the filtrate was concentrated to dryness. 50mL of ethanol and 10mL of 30% sodium hydroxide solution were added to the concentrate, heated to reflux, and stirred for 3 hours, cooled down to room temperature, and 100mL of water was added to age for 30 minutes. It was filtrated and dried under vacuum to abtain 5.0 g of abiraterone, with a yield of 71.5% by molar.

5.0g (0.014mol) of abiraterone, 50mL of dichloromethane, and 3.0g (0.03mol) of triethylamine were added to a reaction flask. The reaction was cooled down to 0~5 °C, and 3.0g (0.03mol) of acetic anhydride was added dropwise and slowly. After the addition was completed, it was heated up to room temperature, reacted at this temperature for 3 hours, and abiraterone was completely reacted, The mixture was filtered, and the organic layer was washed sequentially with 20 mL of water and 20 mL of 1N sodium bicarbonate aqueous solution, concentrated to dryness under reduced pressure, and 20mL of 90% ethanol water was added to the concentrate. The mixture was heated to reflux to allow it dissolved and clear, cooled down to 0~5 °C at a constant speed within two hours, crystallized, filtrated, and dried under vacuum to obtain 5.3g of crude abiraterone acetate, with a yield of 94.5% by molar and a purity of 98.3% by HPLC. The crude product was recrystallized from acetone solvent to obtain 4.6g of abiraterone acetate, with a purity of 99.5%. ESI-MS (m/z): 414 [M+Na]⁺.

### Example 9

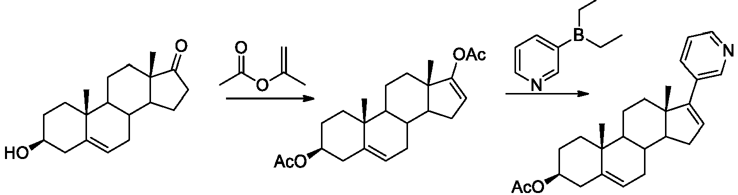

1.44kg (5.0mol) of dehydroepiandrosterone, 86.0g (0.5mol, 0.1equiv) of p-toluenesulfonic acid, and 10.0 L of isopropenyl acetate were added to a reaction flask, and the reaction mixture was heated up to 120 ± 5 °C, and distilled at atmospheric pressure for 2 hours until the volume of the remaining material was about 3L, and the distillation stopped, it continue to be stirred at this temperature for 3 hours. It was cooled down to 0~5 °C, stirred for 30 minutes, filtered, and the filter cake was added to 5L of water, and it was stirred for 30 minutes, filtered, and the filter cake was added to 3L of ethanol, pulped at room temperature for 30 minutes, cooled down to 0~5 °C, stirred for 30 minutes, filtered, and the filter cake was rinsed with a small amount of cold ethanol, dried under vacuum at 35-40 °C to obtain 1.72Kg of 17-acetate androst-5,16-diene-3β-acetate, with a yield of 92.2% by molar and a purity of 99.2% by HPLC.

744.0g (2.0 mol) of 17-acetate androst-5,16-diene-3β-acetate, 308.7g (2.1mol) of diethyl (3-pyridyl) borane, 14.0g (0.02 mol) of PdCl₂(PPh₃)₂ (i.e. the metal catalyst was palladium chloride and ligand was triphenylphosphine), 414.0g (3.0 mol) of potassium carbonate, 4.0L of DMF were added to a reaction flask. After nitrogen substitution, the reaction mixture was heated up to 60 °C and stirred for 5 hours. The reaction was monitored by TLC, and after it was completed, the reaction mixture was cooled down to room temperature, transferred slowly to a 50L reactor containing 20L of water, stirred for 30 minutes, filtered, and the filter cake was added to 10L of dichloromethane to allow it dissolved. It was washed with 2L of 1N hydrochloric acid aqueous solution, 2L of 1N sodium bicarbonate aqueous solution, and 2L of water sequentially. The organic layer was concentrated under reduced pressure to dryness, and 3L of 90% ethanol aqueous solution was added to the concentrate, and it was heated to reflux to allow it dissolved and clear, cooled down to 0~5 °C within 2 hours, crystallized, filtered, and dried under vacuum to obtain 736.5g of crude abiraterone acetate, with a yield of 94.1% by molar and a purity of 98.9% by HPLC. The crude product was recrystallized from acetone solvent to obtain 702.6g of abiraterone acetate, with a purity of 99.8%.

The product was confirmed as abiraterone acetate by mass spectrometry and nuclear magnetic resonance detection.
m.p.:144.8°C-146.3°C; ESI-MS(m/z):414 [M+Na]⁺; 1H NMR (500 MHz, CDCl₃) δ(ppm): 8.932 (s, 1H),8.812 (d, 1H),8.632 (d, 1H), 8.072~8.032 (dd, 1H), 6.561 (s, 1H), 5.386 (d, 1H), 4.483~4.424 (m, 1H), 2.322~2.265 (m, 3H), 2.141~2.093 (m, 2H), 2.043~2.008 (m, 1H), 1.992 (s, 3H), 1.861~1.773 (m, 2H), 1.743~1.642 (m, 3H), 1.601~1.524 (m, 3H), 1.452~1.392 (m, 1H), 1.111~1.013 (m, 2H), 1.052 (s, 3H), 1.042(s, 3H)
¹³C NMR (125 MHz, CDCl₃) δ (ppm): 170.6, 151.8, 148.1, 148.0, 140.1, 133.0, 133.8, 129.3, 123.1, 122.4, 74.0, 57.6, 50.4, 47.4, 36.9, 38.2, 37.0, 35.3, 31.9, 31.6, 30.5, 27.8, 21.5, 20.9, 19.4, 16.7.

### Example 10

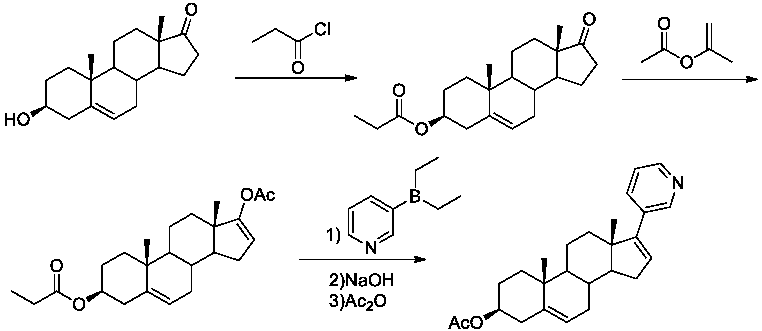

14.4g (0.05mol) of dehydroepiandrosterone, 100mL of dichloromethane, and 6.1g (0.06mol) of triethylamine were added to a reaction flask, and the mixture was stirred until dissolved and clear, cooled down to 0~5 °C, and 4.9g (52.5mmol) of propionyl chloride was added dropwise and slowly. After the addition was completed, the reaction mixture was heated up to room temperature, and stirred at this temperature for 3 hours. The reaction was monitored by TLC, and after it was completed, 50mL of water was added slowly. The water layer was removed, and the organic layer was dried with anhydrous sodium sulfate, filtered, and the filtrate was concentrated to dryness. 0.9g (5.0mmol) of p-toluenesulfonic acid and 100mL of isopropenyl acetate were added to the concentrate, heated up to 120 ± 5 °C, and distilled at atmospheric pressure for 1 hour. Until the volume of the remaining material was about 50mL, the distillation stopped, and it continued to be stirred at this temperature for 2 hours; cooled down to 0~5 °C, stirred for 30 minutes; filtered, and 80mL of water was added to the filter cake, and it was stirred for 30 minutes; filtered, and the filter cake was added to 30mL of ethanol, and it was pulped at room temperature for 30 minutes; cooled down to 0~5 °C, stirred for 30 minutes; filtered, and the filter cake was rinsed with a small amount of cold ethanol, and dried under vacuum at 35-40 °C to obtain 17.7g of 17-acetate androst-5,16-diene-3β-propionate, with a yield of 91.5% by molar and a purity of 99.3% by HPLC.

7.7g (0.02mol) of 17-acetate androst-5,16-diene-3β-propionate, 2.94g (0.02mol) of diethyl (3-pyridyl) borane, 0.02g (0.1mmol) of nickel acetate, 0.02g (0.1mmol) of 2,2-biphyridine, 4.24g (0.02mol) of potassium phosphate, and 50mL of toluene were added to a reaction flask. After nitrogen substitution, the reaction mixture was stirred vigorously, heated to reflux and stirred for 5 hours. The reaction was monitored by TLC, and after it was completed, the reaction solution was filtered while hot, and the filtrate was concentrated to dryness. The concentrate was added to a mixture of 50mL of ethanol and 10mL of 30% sodium hydroxide solution, heated to reflux, stirred for 3 hours, cooled down to room temperature, and 100mL of water was added to age for 30 minutes. It was filtrated and dried under vacuum to abtain 6.1 g of abiraterone, with a yield of 87.6% by molar.

The product was confirmed as abiraterone by mass spectrometry and nuclear magnetic resonance detection.

ESI-MS(m/z):372 [M+Na]⁺; ¹H NMR (500 MHz, CDCl₃) δ (ppm): 8.58 (s, 1H), 8.43 (d, 1H), 7.76~7.74 (d, 1H), 7.34~7.32 (q, 1H), 6.11 (s, 1H), 5.30 (s, 1H), 4.62 (d, 1H), 2.19~2.10 (m, 3H), 2.07~2.02 (m, 3H), 1.77 (m, 1H), 1.69~1.61 (m, 4H), 1.53~1.51 (m, 2H), 1.37~1.35 (m, 2H), 1.01~0.96 (m, 8H).

6.1g (0.017mol) of abiraterone, 50mL of dichloromethane, and 3.0g (0.03mol) of triethylamine were added to a reaction flask. The reaction was cooled down to 0~5 °C, and 3.0g (0.03mol) of acetic anhydride was added dropwise and slowly. After the addition was completed, the reaction mixture was heated up to room temperature, reacted at this temperature for 3 hours, filtered, and the organic layer was washed with 20mL of water and 20mL of 1N sodium bicarbonate aqueous solution sequentially, concentrated to dryness under reduced pressure, and 20mL of 90% ethanol aqueous solution was added to the concentrate, and the reaction solution was heated to reflux to allow it dissoved and clear, cooled down to 0~5 °C within 2 hours, crystallized, filtrated, and dried under vacuum to obtain 6.4g of crude abiraterone acetate, with a yield of 96.8% by molar and a purity of 98.5% by HPLC. The crude product was recrystallized from acetone solvent to obtain 5.8g of abiraterone acetate, with a purity of 99.7%.

### Example 11

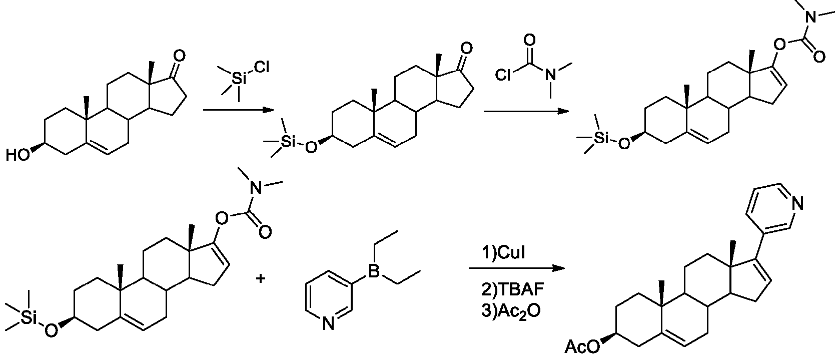

14.4g (0.05mol) of dehydroepiandrosterone, 100mL of dichloromethane, and 6.1g (0.06mol) of triethylamine were added to a reaction flask, and the mixture was stirred until dissolved and clear, cooled down to 0~5 °C, and 5.7g (52.5mmol) of trimethylchlorosilane was added dropwise and slowly. After the addition was completed, the reaction mixture was heated up to room temperature and stirred at this temperature for 4 hours. The reaction was monitored by TLC, and after it was completed, 50mL of water was added. The water layer was removed, and the organic layer was dired with anhydrous sodium sulfate, filtered, and the filtrate was concentrated to dryness. 5.0g (0.05mol) of triethylamine and 100mL of tetrahydrofuran were added to the concentrate, and it was stirred for 10 minutes, cooled down to 0~5 °C, and 3.2g (0.05mol) of dimethylaminoformyl chlorides was added slowly, and it was heated to reflux, and stirred at this temperature for 5 hours. The reaction was monitored by TLC, and after it was completed, it was concentrated under reduced pressure until the volume of the remaining material was about 50mL, and 80mL of water was added, and it was stirred for 30 minutes, filtered, and the filter cake was added to 30mL ethanol, and it was pulped at room temperature for 30 minutes, and the slurry was cooled down to 0~5 °C, stirred for 30 minutes, filtered, and the filter cake was rinsed with a small amount of cold ethanol, and dried under vacuum at 35-40 °C to obtain 18.6g of 17-dimethylaminoformyl ester androst-5,16-diene-3β- trimethylsilyl ester, with a yield of 86.5% by molar and a purity of 98.6% by HPLC.

8.6g (0.02 mol) of dimethylaminoformyl ester androst-5,16-diene-3β-trimethylsilyl ester, 4.41g (0.03mol) of diethyl (3-pyridyl) borane, 0.8g (4.0mmol) of cuprous iodide, 0.7g (4.0mmol) of 1,10-phenanthroline, 2.5g (0.03mol) of sodium bicarbonate, and 50mL of DMSO were added to a reaction flask. After nitrogen replacement, the reaction mixture was stirred vigorously, heated up to 80 degrees, and stirred for 3 hours. The reaction was monitored by TLC, and after it was completed, the reaction solution was cooled down to room temperature, and 200mL of water was added dropwise and slowly, stirred for 30 minutes, filtered, and the filter cake was added to a solution of 50mL THF and 7.8g tetrabutyl ammonium fluoride, and it was stirred at room temperature for 5 hours, and 100mL of water was added to age for 30 minutes. It was filtrated and dried under vacuum to abtain 5.6 g of abiraterone, with a yield of 79.2% by molar.

5.6g (0.016mol) of abiraterone, 50mL of dichloromethane, and 2.0g (0.02mol) of triethylamine were added to a reaction flask. The reaction was cooled down to 0~5 °C, and 2.0g (0.02mol) of acetic anhydride was added dropwise and slowly. After the addition was completed, the reaction solution was heated up to room temperature, reacted at this temperature for 3 hours, filtered, and the organic layer was washed sequentially with 20 mL of water, 20 mL of 1N sodium bicarbonate solution, and then washed with 20 mL of 1N EDTA solution to remove copper ions. The organic layer was concentrated to dryness under reduced pressure, and 20mL of 90% ethanol water was added to the concentrate. The mixture was heated to reflux to allow it dissoved and clear, cooled down to 0~5 °C within 2 hours, crystallized, filtrated, and dried under vacuum to obtain 6.0g of crude abiraterone acetate, with a yield of 95.1% by molar and a purity of 97.0% by HPLC. The crude product was recrystallized from acetone solvent to obtain 5.3g of abiraterone acetate, with a purity of 99.2%. ESI-MS (m/z): 414 [M+Na]⁺.

### Example 12

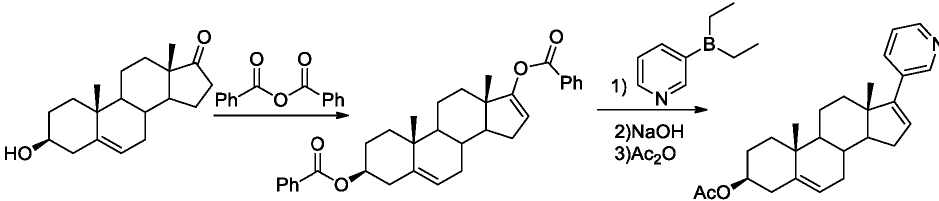

14.4g (0.05mol) of dehydroepiandrosterone, 11.2g (0.1mol) of potassium tert-butoxide, and 100mL of tetrahydrofuran were added to a reaction flask, and the mixture was stirred for 10 minutes, cooled down to 0~5 °C, and 22.6g (0.1mol) of benzoic anhydride was added slowly. The reaction mixture was heated up to 60-65 °C, and stirred at this temperature for 3 hours. The reaction was monitored by TLC, and after it was completed, it was concentrated under reduced pressure until the volume of the remaining material was about 50mL, and 80mL of water was added, and it was stirred for 30 minutes, filtrated, and the filter cake was added to 50mL of water, stirred for 30 minutes, filtrated. and the filter cake was then added to 30mL of ethanol, and it was pulped at room temperature for 30 minutes, cooled down to 0~5 °C, stirred for 30 minutes, filtrated, and the filter cake was rinsed with a small amount of cold ethanol, and dried under vacuum at 35-40 °C to obtain 22.6g of 17-benzoate androst-5,16-diene-3β-benzoate, with a yield of 91.2% by molar and a purity of 98.6% by HPLC.

9.9g (0.02 mol) of 17-benzoate androst-5,16-diene-3β-benzoate, 4.41g (0.03 mol) of diethyl (3-pyridyl) borane, 1.38g (2.0mmol) of tricyclohexylphosphine nickel chloride (i.e. the metal catalyst was nickel chloride and ligand was tricyclohexylphosphine), 2.4g (0.03 mol) of lithium tert-butoxide, and 60mL of acetonitrile were added to a reaction flask. After nitrogen replacement, the reaction mixture was stirred vigorously, heated to reflux, and stirred for 6 hours. The reaction was monitored by TLC, and after it was completed, the reaction solution was filtered while hot, and the filtrate was concentrated to dryness. The concentrate was added to a mixture of 50mL of ethanol and 10mL of 30% sodium hydroxide solution, and it was heated to reflux, stirred for 3 hours, cooled down to room temperature, and 100mL of water was added to age for 30 minutes. It was filtrated and dried under vacuum to abtain 5.7 g of abiraterone, with a yield of 82.9% by molar.

5.7g (0.016mol) of abiraterone, 50mL of dichloromethane, and 2.0g (0.02mol) of triethylamine were added to a reaction flask. The reaction was cooled down to 0~5 °C, and 2.0g (0.02mol) of acetic anhydride was added dropwise and slowly. After the addition was completed, the reaction mixture was heated up to room temperature, reacted at this temperature for 3 hours, filtered, and the organic layer was washed with 20mL of water and 20mL of 1N sodium bicarbonate aqueous solution sequentially, concentrated to dryness under reduced pressure. The concentrate was added to a 20mL of 90% ethanol aqueous solution, and it was heated to reflux to allow it dissoved and clear, cooled down to 0~5 °C for 2 hours, crystallized, filtered, and dried under vacuum to obtain 6.1g of crude abiraterone acetate, with a yield of 97.5% by molar and a purity of 99.0% by HPLC. The crude product was recrystallized from acetone to obtain 5.4g of abiraterone acetate, with a purity of 99.7%. ESI-MS (m/z): 414 [M+Na]⁺.

### Example 13

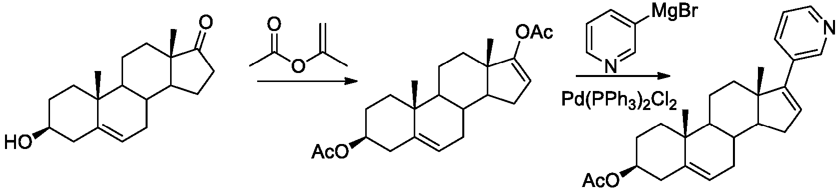

1.44kg (5.0mol) of dehydroepiandrosterone, 86.0g (0.5mol, 0.1equiv) of p-toluenesulfonic acid, and 10.0 L of isopropenyl acetate were added to a reaction flask. The reaction mixture was heated up to 120 ± 5 °C, and distilled at atmospheric pressure for 2 hours until the volume of the remaining material was about 3L, and the distillation stopped, and it continue to be stirred at this temperature for 3 hours, cooled down to 0~5 °C, stirred for 30 minutes, filtered, and the filter cake was added to 5L of water, and it was stirred for 30 minutes, filtered, and the filter cake was added to 3L of ethanol, and it was pulped at room temperature for 30 minutes, cooled down to 0~5 °C, stirred for 30 minutes, filtered, and the filter cake was rinsed with a small amount of cold ethanol, and dried under vacuum at 35-40 °C to obtain 1.72Kg of 17-acetate androst-5,16-diene-3β-acetate, with a yield of 92.2% by molar and a purity of 99.2% by HPLC.

3-bromopyridine (1 equivalent, 7.5 mol, 1185g) and 2.7L of tetrahydrofuran were added to a three necked reaction flask with stir, and the reaction solution was cooled down to -20 °C, and a THF solution of isopropyl magnesium chloride and lithium chloride (1.3 M, 1.0 equivalent, 7.5 mol, 5.8 L) was added dropwise and slowly, and the reaction mixture was heated up to room temperature, stirred for more than 6 hours to obtain pyridine Grignard reagent (concentration was about 1mol/L).

744.0g (2.0 mol) of 17-acetate androst-5,16-diene-3β-acetate, 164g (2.0 mol) of sodium acetate, 14.1g (0.02 mol) of PdCl₂(PPh₃)₂ (equivalent to that the metal catalyst was PdCl₂ and the ligand was PPh₃), and 8.0L of tetrahydrofuran were added to a reaction flask, and the reaction was stirred until dissoved and clear, cooled down to -30 °C, and 2.0L (2.0 mol) of pyridine Grignard tetrahydrofuran solution was added dropwise and slowly. After the addition was completed, the reaction mixture was heated to room temperature and stirred for 5 hours. The reaction was monitored by TLC, and after it was completed, saturated ammonium chloride solution was added dropwise and slowly to quench, and it was concentrated to dryness under reduced pressure. Dichloromethane was added to dissove the concentrate, and it was filtered, and the filtrate was washed with 2L of 1N hydrochloric acid aqueous solution, 2L of 1N sodium bicarbonate aqueous solution, 2L of 1N EDTA disodium solution, and 2L of water sequentially. The organic layer was concentrated to dryness under reduced pressure, and 3L of 90% ethanol aqueous solution was added to the concentrate. The mixture was heated to reflux to allow it dissoved and clear, cooled down to 0~5 °C within two hours, crystallized, filtrated, and dried under vacuum to obtain 716.6g of crude abiraterone acetate, with a yield of 91.6% by molar and a purity of 98.2% by HPLC, and the impurities of abiraterone accounted for 0.6%. The crude product was recrystallized from acetone to obtain 667.2g of abiraterone acetate, with a purity of 99.8%.

The product was confirmed as abiraterone acetate by mass spectrometry and nuclear magnetic resonance detection.
ESI-MS(m/z):414 [M+Na]⁺;
¹H NMR (500 MHz, CDCl₃) δ (ppm): 8.93 (s, 1H), 8.82 (d, 1H), 8.64 (d, _{1H),} 8.07-8.04 (dd, 1H), 6.56 (s, 1H), 5.39 (d, 1H), 4.48-4.42 (m, 1H), 2.32-2.27 (m, 3H), 2.14-2.09 (m, 2H), 2.04-2.01 (m, 1H), 1.99 (s, 3H), 1.86-1.77 (m, 2H), 1.74-1.64 (m, 3H), 1.60-1.52 (m, 3H), 1.45~1.39 (m, 3H) 1H), 1.11~1.01 (m, 2H), 1.05 (s, 3H), 1.04 (s, 3H). ¹³C NMR (125 MHz, CDCl₃) δ (ppm): 170.6, 151.8, 148.1, 148.0, 140.1, 133.0, 133.8, 129.3, 123.1, 122.4, 74.0, 57.6, 50.4, 47.4, 36.9, 38.2, 37.0, 35.3, 31.9, 31.6, 30.5, 27.8, 21.5, 20.9, 19.4, 16.7.

The function of ligands and additives is to increase reaction activity, but the reaction can still proceed without the addition of a ligand and an additive, while the content of the target product in the reaction solution decreases. The reaction conditions of step 2 in Example 1 was used as a model experiment, under the same experimental conditions (reaction substrate and dosage thereof, catalyst (if present in the reaction system) and dosage thereof, ligand (if present in the reaction system) and dosage thereof, additive (if present in the reaction system) and dosage thereof, solvent, reaction temperature, etc. were same as those in step 2 in Example 1). The reaction results without the addition of ligands and/or additives are shown in Table 1 below.

**Table 1**

| Examples | Catalysts | Ligands | Additives | Purity of coupling reaction solution | Yield of crude |
|---|---|---|---|---|---|
| 1-1 | PdCl₂ | PPh₃ | Sodium acetate | 96.3% | 91.6% |
| 1-2 | PdCl₂ | / | Sodium acetate | 72% | 55.6% |
| 1-3 | PdCl₂ | PPh₃ | / | 84.2% | 68.9% |
| 1-4 | / | PPh₃ | Sodium acetate | 0 | 0 |

It can be seen from Table 1 that there were no ligands in the reaction system of Example 1-2, and the reaction was monitored by TLC, and after it was completed, HPLC was used to detect the reaction solution, and the content of the target product was about 72.0%. There were no additives in the reaction system of Example 1-3, and the reaction was monitored by TLC, and after it was completed, HPLC was used to detect the reaction solution, and the content of the target product was about 84.2%, which was significantly lower than the content of the target product in the reaction solution of Example 1-1. There was no catalyst in the reaction system of Example 1-4, and the coupling reaction couldn't occur.

### Example 14

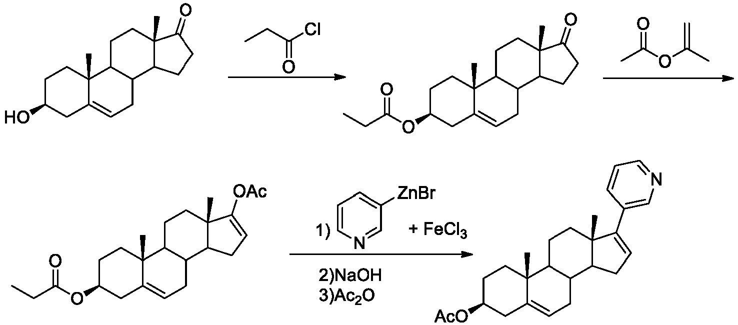

14.4g (0.05mol) of dehydroepiandrosterone, 100mL of dichloromethane, and 6.1g (0.06mol) of triethylamine were added to a reaction flask, and the mixture was stirred until dissoved and clear, cooled down to 0~5 °C, and 4.9g (52.5mmol) of propionyl chloride was added dropwise and slowly. After the addition was completed, the reaction mixture was heated up to room temperature, and stirred at this temperature for 3 hours. The reaction was monitored by TLC, and after it was completed, 50mL of water was added slowly to the reaction solution. Let it stand and layer, and the water layer was removed, and the organic layer was dried with anhydrous sodium sulfate, filtered, and the filtrate was concentrated to dryness. 0.9g (5.0mmol) of p-toluenesulfonic acid and 100mL of isopropenyl acetate were added to the concentrate, and it was heated up to 120 ± 5 °C, and distilled at atmospheric pressure for 1 hour. Until the volume of the remaining material was about 50mL, the distillation stopped, and it continued to be stirred for 2 hours at this temperature. The reaction mixture was cooled down to 0~5 °C, stirred for 30 minutes, filtered, and the filter cake was added to 80mL of water, and it was stirred for 30 minutes, filtered, and the filter cake was added to 30mL of ethanol, and it was pulped at room temperature for 30 minutes, cooled down to 0~5 °C, stirred for 30 minutes, filtered, and the filter cake was rinsed with a small amount of cold ethanol, dried under vacuum at 35-40 °C to obtain 17.7g of 17-acetate androst-5,16-diene-3β-propionate, with a yield of 91.5% by molar and a purity of 99.3% by HPLC.

200ml of pyridine Grignard reagent (concentration was about 1 mol/L) prepared according to Example 1 was added to a three necked flask. It was cooled down to 0 °C, and 13.0g (0.2mol) of zinc powder was added while stirred. After the addition was complete, it was heated up to room temperature and continued to be stirred for 3 hours to obtain pyridine zinc reagent, which was ready for use.

7.7g (0.02 mol) of 17-acetate androst-5,16-diene-3β-propionate, 50mL of 2-methyltetrahydrofuran, 0.64g (0.004mol) of anhydrous ferric chloride, 2.3g (0.02 mol) of TMEDA, 5.88g (0.06mol) of potassium acetate were added to a reaction flask, and 30ml (0.03 mol) of zinc pyridine reagent was added dropwise at room temperature. It was stirred vigorously, heated up to 50 °C, and reacted for 5 hours. The reaction was monitored by TLC, and after it was completed, it was filtered while hot, and the filtrate was concentrated to dryness. 50mL of ethanol and 10mL of 30% sodium hydroxide solution were added to the concentrate, and it was heated up to reflux, refluxed for 3 hours, cooled down to room temperature, and 100mL of water was added to age for 30 minutes. It was filtered, and the filter cake was dried under vacuum to obtain 5.7g of abiraterone, with a yield of 81.7% by molar.

The product was confirmed as abiraterone by mass spectrometry and nuclear magnetic hydrogen spectroscopy.

ESI-MS (m/z): 372 [M+Na]⁺.

¹H NMR (500 MHz, CDCl₃) δ (ppm): 8.58 (s, 1H), 8.43 (d, 1H), 7.76~7.74 (d, 1H), 7.34~7.32 (q, 1H), 6.11 (s, 1H), 5.30 (s, 1H), 4.62 (d, 1H), 2.19~2.10 (m, 3H), 2.07~2.02 (m, 3H), 1.77 (m, 1H), 1.69~1.61 (m, 4H), 1.53~1.51 (m, 2H), 1.37~1.35 (m, 2H), 1.01~0.96 (m, 8H).

5.7g (0.016mol) of abiraterone, 50mL of dichloromethane, and 3.0g (0.03mol) of triethylamine were added to a reaction flask. The reaction was cooled down to 0~5 °C, and 3.0g (0.03mol) of acetic anhydride was added dropwise and slowly. After the addition was completed, the reaction mixture was heated up to room temperature, reacted at this temperature for 3 hours, filtered to remove insoluble substances, and the filtrate was washed sequentially with 20 mL of water and 20 mL of 1N sodium bicarbonate aqueous solution. The organic layer was concentrated to dryness under reduced pressure, and 20mL of 90% ethanol aqueous solution was added to the concentrate. The mixture was heated to reflux to allow it dissolve and clear, cooled down to 0~5 °C within 2 hours, crystallized, filtrated, and the filter cake was dried under vacuum to obtain 5.9g of crude abiraterone acetate, with a yield of 92.5% by molar and a purity of 97.8% by HPLC. The crude product was recrystallized from acetone to obtain 5.1g of abiraterone acetate, with a purity of 99.5%. ESI-MS (m/z): 414 [M+Na]⁺.

### Example 15

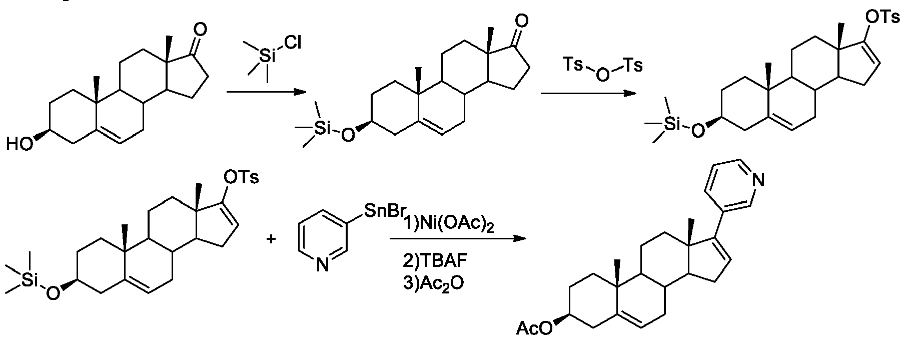

14.4g (0.05mol) of dehydroepiandrosterone, 100mL of dichloromethane, and 6.1g (0.06mol) of triethylamine were added to a reaction flask, and the mixture was stirred until dissolved and clear, cooled down to 0~5 °C, and 5.7g (52.5mmol) of trimethylchlorosilane was added dropwise and slowly. After the addition was completed, the reaction mixture was heated up to room temperature, stirred at this temperature for 4 hours. The reaction was monitored by TLC, and after it was completed, 50mL of water was added to the reaction solution. Let it stand and layer, and the water layer was removed, and the organic layer was dried with anhydrous sodium sulfate, filtered, and the filtrate was concentrated to dryness. 5.6g (0.05mol) of potassium tert-butoxide and 100mL of tetrahydrofuran were added to the concentrate, and the mixture was stirred for 10 minutes, cooled down to 0~5 °C, 16.3g (0.05mol) of p-toluenesulfonic anhydride was added, and it was heated up to 60-65 °C, and stirred at this temperature for 5 hours. The reaction was monitored by TLC, and after it was completed, the reaction solution was concentrated under reduced pressure until the volume of the remaining material was about 50mL, and 80mL of water was added, and it was stirred for 30 minutes, filtered, and the filter cake was added to 30mL of ethanol, and it was pulped at room temperature for 30 minutes. The slurry was cooled down to 0~5 °C, stirred for 30 minutes, filtered, and the filter cake was washed with a small amount of cold ethanol, dried under vacumn at 35-40 °C to obtain 21.2g of 17-p-tosylate androst-5,16-diene-3β-trimethylsilyl ester, with a yield of 82.6% by molar and a purity of 98.3% by HPLC.

200ml of pyridine Grignard reagent (concentration was about 1 mol/L) prepared according to Example 1 was added to a three necked flask. It was cooled down to 0 °C, and 52.0g (0.2mol) of anhydrous tin chloride was added while stirred. After the addition was complete, it was heated up to room temperature and continued to be stirred for 3 hours to obtain pyridine tin reagent, which was ready for use.

10.3g (0.02 mol) of 17-p-tosylate androst-5,16-diene-3β-trimethylsilyl ester, 0.35g (0.002 mol) of nickel acetate, 0.56g (0.002 mol) of tricyclohexylphosphine, 2.12g (0.02 mol) of sodium carbonate, and 50mL of ethylene glycol dimethyl ether were added to a reaction flask, and the mixture was stirred evenly, and then 20ml of pyridine tin reagent was added at room temperature. The reaction mixture was heated up to 40 °C and stirred for 5 hours. The reaction was monitored by TLC, and after it was completed, it was cooled down to room temperature, filtered, and the filtrate was concentrated to dryness. The concentrate was added to 50mL of ethanol and 10mL of 30% sodium hydroxide solution, and the mixture was heated up to reflux, stirred for 5 hours, cooled down to room temperature, and100mL of drinking water was added to age for 30 minutes. It was filtered, and the filter cake was dried under vacuum to obtain 4.6g of abiraterone, with a yield of 66.1% by molar.

4.6g (0.013mol) of abiraterone, 50mL of dichloromethane, and 2.0g (0.02mol) of triethylamine were added to a reaction flask. The mixture was cooled down to 0~5 °C, and 2.0g (0.02mol) of acetic anhydride was added dropwise and slowly. After the addition was completed, it was heated up to room temperature, reacted at this temperature for 3 hours, filtered to remove insoluble substances, and the filtrate was washed with 20 mL of water and 20 mL of 1N sodium bicarbonate aqueous solution sequentially. The organic layer was concentrated under reduced pressure to dryness. 20mL of 90% ethanol water was added to the concentrate. The mixture was heated to reflux to allow it dissolved and clear, cooled down to 0~5 °C within two hours, crystallized, filtrated, and the filter cake was dried under vacuum to obtain 4.8g of crude abiraterone acetate, with a yield of 94.4% by molar and a purity of 98.0% by HPLC. The crude product was recrystallized from acetone to obtain 4.0g of abiraterone acetate, with a purity of 99.5%. ESI-MS (m/z): 414 [M+Na]⁺.

### Example 16

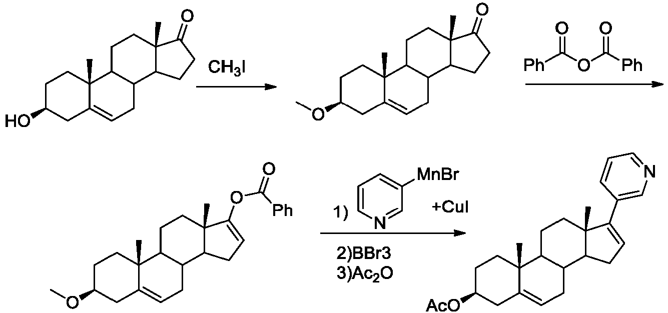

14.4g (0.05mol) of dehydroepiandrosterone, 11.2g (0.1mol) of potassium tert-butoxide, and 50mL of DMF were added to a reaction flask, and the mixture was stirred for 10 minutes, cooled down to 0~5 °C, and 7.1g (0.05mol) of iodomethane was added slowly. The reaction mixture was heated up to 40 °C, stirred overnight, and 200ml of water was added, and it was filtered, and the filter cake was dried directly, dissoved in 100mL of tetrahydrofuran, and 12.4g (0.55mol) of benzoic anhydride was added. The reaction was refluxed, and stirred at room temperature for 5 hours. The reaction was monitored by TLC, and after it was completed, the reaction solution was concentrated under reduced pressure until the volume of the remaining material was about 50mL, and 80mL of water was added, and it was stirred for 30 minutes, filtered, and the filter cake was added to 50mL of water, stirred for 30 minutes, filtered, and the filter cake was added to 30mL of ethanol, and it was pulped at room temperature for 30 minutes. The slurry was cooled down to 0~5 °C, stirred for 30 minutes, filtered, and the filter cake was washed with a small amount of cold ethanol. It was dried under vacuum at 35-40 °C to obtain 16.8g of 17-benzoate androstane-5,16-diene-3β-methyl ether, with a yield of 83.0% by molar and a purity of 97.5% by HPLC.

200ml of pyridine Grignard reagent (concentration was about 1 mol/L) prepared according to Example 1 was added to a three necked flask. It was cooled down to 0 °C, and 100ml of toluene was added, and 25.2g (0.2mol) of anhydrous manganese chloride was added while stirred. After the addition was complete, the reaction mixture was heated up to room temperature and stirred for 3 hours to obtain pyridine manganese reagent, which was ready for used.

8.1g (0.02 mol) of 17-benzoate androst-5,16-diene-3β-methyl ether, 0.38g (0.002mol) of cuprous iodide, 0.23g (0.002mol) of L-proline, 6.4g (0.03 mol) of anhydrous potassium phosphate, 40mL of tetrahydrofuran were added to a reaction flask. 33ml of pyridine manganese reagent was added dropwise and slowly, and it was stirred vigorously. The reaction mixture was heated up to 50 °C and stirred for 6 hours. The reaction was monitored by TLC, and after it was completed, it was filtered while hot, and the filtrate was concentrated to dryness, and then steamed once with dichloromethane. The concentrate was dissolved in 50mL of dichloromethane, and it was cooled down to 0 °C, and 5mL of boron tribromide was added, and it was stirred for 3 hours, and 10mL of methanol was added dropwise and slowly to quench, and 100mL of water was added. Let it stand and layer to obtain the organic layer, and it was concentrated to dryness, crystallized with 60% ethanol aqueous solution, filtered, and dried under vacuum to obtain 4.9g of abiraterone, with a yield of 70.2% by molar.

4.9g (0.014mol) of abiraterone, 50mL of dichloromethane, and 3.0g (0.03mol) of triethylamine were added to a reaction flask. The mixture was cooled down to 0~5 °C, and 3.0g (0.03mol) of acetic anhydride was added dropwise and slowly. After the addition was complete, the mixture was heated up to room temperature, reacted at this temperature for 3 hours, filtered to remove the insoluble substances, and the filtrate was washed with 20mL of water and 20mL of 1N sodium bicarbonate aqueous solution sequentially. It was concentrated to dryness under reduced pressure, and the concentrate was added to 20mL of 90% ethanol water. It was heated to reflux to allow it dissolved and clear, cooled down to 0~5 °C within two hours, crystallized, filtered, and dried under vacuum to obtain 5.2g of crude abiraterone acetate, with a yield of 95.0% by molar and a purity of 98.0% by HPLC. The crude product was recrystallized from acetone solvent to obtain 4.7g of abiraterone acetate, with a purity of 99.6%. ESI-MS (m/z): 414 [M+Na]⁺.

All references mentioned in the present invention are cited as references in this application, as if each reference were cited individually. In addition, it should be understood that after reading the above teaching content of the present invention, those skilled in the art can make various changes or modifications to the present invention, and these equivalent forms also fall within the scope defined by the appended claims of the present application.

## Claims

1. A preparation method for abiraterone or an intermediate thereof, wherein the method comprises the steps of: (i) in an organic solvent, in the presence of a metal catalyst, ligand, and reducing agent, reacting a compound of formula II with a compound of formula III to obtain a compound of formula I;
the reaction formula is as follows: 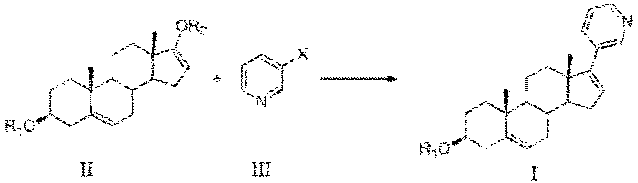
R₁ is selected from the group consisting of: hydrogen, C₁-C₆ alkyl, phenyl, benzyl, C₁-C₆ acyl, substituted or unsubstituted benzoyl, methylsulfonyl, substituted or unsubstituted benzenesulfonyl, or C₁-C₂ trialkylsilyl;
R₂ is selected from the group consisting of: C₁-C₆ acyl, substituted or unsubstituted benzoyl, methylsulfonyl, substituted or unsubstituted benzenesulfonyl, dimethylamino acyl or diethylaminoacyl, or diphenylphosphinyl acyl; and
X is fluorine, chlorine, bromine or iodine;
wherein, the term "substituted" refers to that one or more hydrogen atoms on a group is independently substituted by a group selected from the group consisting of: halogen, C₁-C₄ alkyl, C₁-C₄ haloalkyl, ether, and nitro;
the metal catalyst is selected from the group consisting of: palladium salt, copper salt, iron salt, cobalt salt, nickel salt, or combinations thereof; and
the ligand is selected from the group consisting of: a phosphorus-containing ligand, amino acid ligand, nitrogen-containing ligand, or combinations thereof,
the reducing agent is selected from the group consisting of: diboron reagent, zinc powder, copper powder, iron powder, magnesium powder, manganese powder, tin powder, samarium powder, indium powder, or combinations thereof.

2. The preparation method according to claim 1, wherein the R₁ is acetyl.

3. The preparation method according to claim 1, wherein the step (i) has one or more features selected from the group consisting of:
the molar ratio of compound of formula II to compound of formula III is 1: 1~4,
the molar ratio of compound of formula II to the metal catalyst is 1: 0.005~0.3,
the molar ratio of compound of formula II to the ligand is 1: 0.005~0.3, and
the molar ratio of compound of formula II to the reducing agent is 1: 1~4.

4. The preparation method according to claim 3, wherein the step (i) has one or more features selected from the group consisting of:
the molar ratio of compound of formula II to compound of formula III is 1: 1.5~3;
the molar ratio of compound of formula II to metal catalyst is 1: 0.01~0.2;
the molar ratio of compound of formula II to the ligand is 1: 0.01~0.2; and/or the molar ratio of compound of formula II to the reducing agent is 1: 1.5~3.

5. The preparation method according to claim 1, wherein the step (i) has one or more features selected from the group consisting of:
the metal catalyst is selected from the group consisting of: bis triphenylphosphine palladium chloride, palladium acetate, palladium chloride, cuprous iodide, copper acetate, ferric chloride, acetylacetonate iron, cobalt chloride, acetylacetonate cobalt, nickel acetate, nickel chloride, tricyclohexylphosphine nickel chloride, or combinations thereof;
the ligand is selected from the group consisting of: triphenylphosphine, tricyclohexylphosphine, L-proline, pyridine, 2,2- biphyridine, 1,10-phenanthroline, or derivatives thereof;
the reducing agent is selected from the group consisting of: diboron reagent, zinc powder, copper powder, iron powder, magnesium powder, manganese powder, tin powder, samarium powder, indium powder, or combinations thereof; and/or
the organic solvent is selected from the group consisting of: tetrahydrofuran, toluene, acetonitrile, N,N-dimethylformamide, N,N-dimethylacetamide, N-methylpyrrolidone, dimethyl sulfoxide, or combinations thereof.

6. The preparation method according to claim 1, wherein in step (i), the reaction temperature is 20-120 °C, preferably 40-100 °C.

7. The preparation method according to claim 1, wherein the method further comprises the steps of: (i-a) reacting a compound of formula IV with an acylation reagent to obtain a compound of formula II;
the reaction formula is as follows: 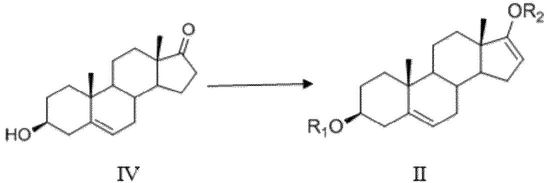
wherein, the acylation reagent used to obtain R₁ is selected from the group consisting of: R₁-halogen, R₁-O-R₁, isopropenyl acetate, or combinations thereof; and
the acylation reagent used to obtain R₂ is selected from the group consisting of: R₂-halogen, R₂-O-R₂, isopropenyl acetate, or combinations thereof.

8. The preparation method according to claim 7, wherein the acylation reagent used to obtain R₁ is selected from the group consisting of: C₁-C₆ alkyl acyl chloride, substituted or unsubstituted benzoic anhydride, isopropenyl acetate, trimethylchlorosilane, or combinations thereof.

9. The preparation method according to claim 7, wherein the acylation reagent used to obtain R₂ is selected from the group consisting of: isopropenyl acetate, substituted or unsubstituted benzoic anhydride, substituted or unsubstituted benzenesulfonic anhydride, or combinations thereof.

10. A preparation method for abiraterone acetate, wherein the method comprises the steps of:
(i) the compound of formula I is prepared by the method according to any one of claims 1~9;
(ii) in an organic solvent, the compound of formula I undergoes a hydrolysis reaction with sodium hydroxide solution to obtain abiraterone; and
(iii) abiraterone undergoes an esterification reaction with acetic anhydride to obtain abiraterone acetate.

11. A preparation method for abiraterone acetate or an intermediate thereof, wherein the preparation method comprises the steps of: (1) in a solvent, in the presence of a metal catalyst, ligand, and electrolyte, under the constant current, reacting the compound of formula II with the compound of formula III to obtain the compound of formula I, and the reaction formula is as follows: 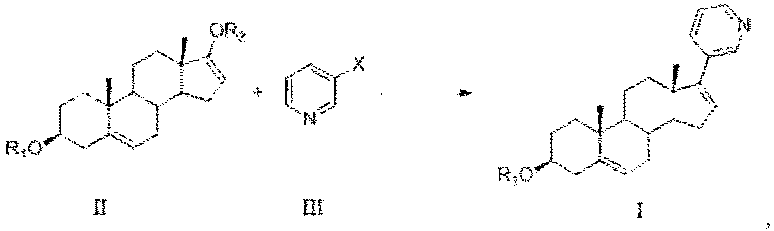
wherein, R₁ is selected from hydrogen, C₁-C₆ alkyl, phenyl, benzyl, C₁-C₆ acyl, substituted or unsubstituted benzoyl, methylsulfonyl, substituted or unsubstituted benzenesulfonyl, or C₁-C₂ trialkylsilyl or dialkylarylsilyl, and the alkyl group in dialkylarylsilyl is a C₁-C₃ alkyl;
R₂ is selected from C₁-C₆ acyl, substituted or unsubstituted benzoyl, methylsulfonyl, substituted or unsubstituted benzenesulfonyl, dimethylamino acyl, diethylamino acyl, or diphenylphosphinyl acyl;
the term "substituted" refers to that one or more hydrogen atoms on a group are independently substituted by a group selected from the group consisting of:
halogen, C₁-C₄ alkyl or C₁-C₄ haloalkyl, ether, nitro;
X is fluorine, chlorine, bromine or iodine;
the metal catalyst is selected from palladium salt, copper salt, cobalt salt, nickel salt, or combinations thereof; and
the ligand is selected from a phosphorus-containing ligand, amino acid containing ligand, pyridine-containing ligand, or combinations thereof.

12. The preparation method according to claim 11, wherein R₁ is acetyl, and the compound of formula I is abiraterone acetate.

13. The preparation method according to claim 11, wherein step (1) has one or more features selected from the following:
(i) the molar ratio of the compound of formula II to the compound of formula III is 1: 1~4, preferably 1: 1~3, more preferably 1: 1.5~2.2, such as 1: 1.8 or 1: 2.0;
(ii) the molar ratio of the compound of formula II to the metal catalyst is 1: 0.005~0.2, preferably 1: 0.01~0.1, more preferably 1: 0.03~0.07, such as 1: 0.04, 1: 0.005 or 1: 0.006;
(iii) the molar ratio of the compound of formula II to the ligand is 1: 0.005~0.3, preferably 1: 0.01~0.2, more preferably 1 :0.05~0.12, such as 1: 0.06, 1: 0.08, 1: 0.10;
(iv) the molar volume ratio of electrolyte to reaction solution is 0.1~0.5 mol/L.

14. The preparation method according to claim 11, wherein in step (1), the constant current is 0.05A~1.0A, more preferably 0.1A~0.5A, and/or
the current density is 0.01~0.2A/cm², more preferably 0.02~0.1A/cm²; and/or
the reaction temperature is 0 °C~60 °C, more preferably 10 °C~50 °C.

15. The preparation method according to claim 11, wherein step (1) has one or more features selected from the following:
(i) the metal catalyst is selected from bis triphenylphosphine palladium chloride, palladium acetate, palladium chloride, palladium trifluoromethanesulfonate, cuprous iodide, copper acetate, copper chloride, cobalt chloride, cobalt acetylacetonate, cobalt acetate, cobalt sulfate, nickel acetate, tricyclohexylphosphine nickel chloride, or combinations thereof,
(ii) the ligand is selected from triphenylphosphine, tricyclohexylphosphine, tri tert-butylphosphine, L-proline, alanine, methionine, pyridine, 2,2-biphyridine, 1,10-phenanthroline, or derivatives thereof;
(iii) the electrolyte is selected from tetraethyl ammonium perchlorate, tetraethyl ammonium p-toluenesulfonate, tetrabutyl ammonium acetate, tetrabutyl ammonium hexafluorophosphate, tetrabutyl ammonium tetrafluoroborate, tetraethyl ammonium tetrafluoroborate, tetraethyl ammonium hexafluorophosphate, or combinations thereof;
(iv) the solvent used in the reaction is selected from acetonitrile, N,N-dimethylformamide, N,N-dimethylacetamide, N-methylpyrrolidone, dimethyl sulfoxide, or combinations thereof.

16. The preparation method according to claim 11, wherein,
R₂ in the compound of formula II is acetyl, X in the compound of formula III is bromine, the metal catalyst is PdCl₂(PPh₃)₂, and the ligand is bipyridine, or
R₂ in the compound of formula II is acetyl, X in the compound of formula III is iodine, the metal catalyst is nickel acetate, and the ligand is tricyclohexylphosphine, or
R₂ in the compound of formula II is p-toluenesulfonyl, X in the compound of formula III is chlorine, the metal catalyst is cuprous iodide, and the ligand is L-proline, or
R₂ in the compound of formula II is benzoyl, X in the compound of formula III is fluorine, the metal catalyst is nickel chloride, and the ligand is 1,10-phenanthroline.

17. The preparation method according to claim 11, wherein the preparation method further comprises reacting the compound of formula IV with an acylation reagent to obtain the compound of formula II;
the reaction formula is as follows: 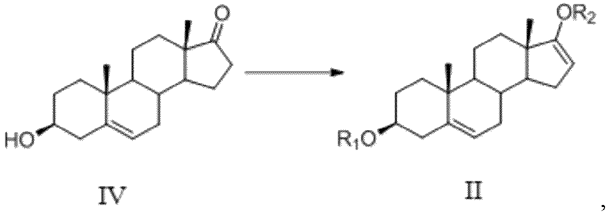
wherein the acylation reagent used to obtain R₁ is selected from R₁-halogen, R₁-O-R₁, or 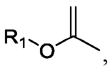
the acylation reagent used to obtain R₂ is selected from R₂-halogen, R₂-O-R₂, or

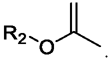

18. The preparation method according to claim 17, wherein the acylation reagent used to obtain R₁ is selected from C₁-C₆ alkyl acyl chloride, substituted or unsubstituted benzoic anhydride, isopropenyl acetate, or trimethylchlorosilane, and/or
the acylation reagent used to obtain R₂ is selected from isopropenyl acetate, substituted or unsubstituted benzoic anhydride, or substituted or unsubstituted benzenesulfonic anhydride.

19. A preparation method for abiraterone, wherein the preparation method further comprises the steps of: (2) daprotecting the compound of formula I prepared by the preparation method according to any one of claims 11 to 18 to obtain abiraterone, and the reaction formula is as follows:

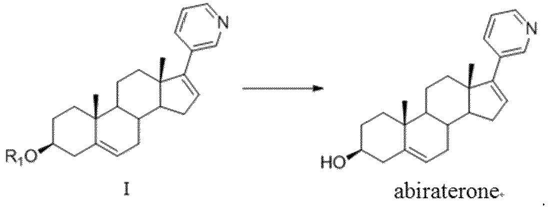

20. A preparation method for abiraterone acetate, wherein the preparation method comprises the steps of: (3) reacting abiraterone prepared by the preparation method according to claim 19 with acetic anhydride to obtain abiraterone acetate, and the reaction formula is as follows:

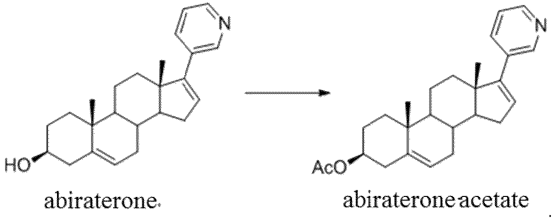

21. A preparation method for abiraterone acetate or an intermediate thereof, wherein the method comprises the steps of: (1) in a solvent, in the presence of a metal catalyst, ligand, and base, reacting a compound of formula II with a compound of formula III-1 to obtain a compound of formula I,
the reaction formula is as follows: 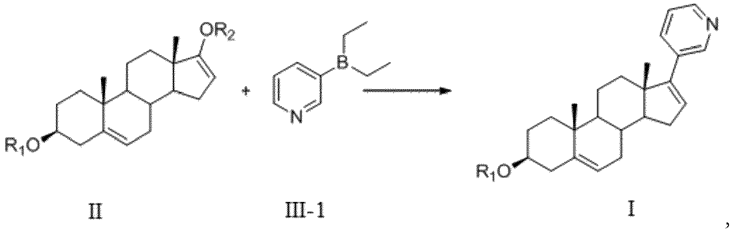
wherein, R₁ is selected from hydrogen, C₁-C₆ alkyl, phenyl, benzyl, C₁-C₆ acyl, substituted or unsubstituted benzoyl, trialkylsilyl or dialkylarylsilyl, and the alkyl in trialkylsilyl and dialkylarylsilyl is C₁-C₃ alkyl,
R₂ is selected from C₁-C₆ acyl, substituted or unsubstituted benzoyl, dimethylamino acyl, diethylamino acyl, or diphenylphosphinyl acyl;
the term "substituted" refers to the one or more hydrogen atoms on a group are independently substituted by a group selected from the group consisting of:
halogen, C₁-C₄ alkyl, C₁-C₄ haloalkyl, ether, nitro,
the metal catalyst is selected from palladium salt, copper salt, iron salt, cobalt salt, nickel salt, or combinations thereof;
the ligand is selected from a phosphorus-containing ligand, amino acid ligand, nitrogen-containing ligand, or combinations thereof.

22. The preparation method according to claim 21, wherein R₁ is selected from C₁-C₆ acyl, a substituted or unsubstituted benzoyl, or trialkylsilyl, more preferably acetyl, and/or
R₂ is selected from C₁-C₆ acyl, substituted or unsubstituted benzoyl, or dimethylaminoformyl, more preferably acetyl.

23. The preparation method according to claim 21, wherein step (1) has one or more features selected from the group consisting of:
the molar ratio of the compound of formula II to the compound of formula III-1 is 1: 1~3, preferably 1: 1~1.5;
the molar ratio of the compound of formula II to the metal catalyst is 1: 0.005~0.3, preferably 1: 0.01~0.2;
the molar ratio of the compound of formula II to the ligand is 1: 0.005~0.3, preferably 1: 0.01~0.2;
the molar ratio of the compound of formula II to the base is 1: 1~3, preferably 1: 1~2.

24. The preparation method according to claim 21, wherein step (1) has one or more features selected from the group consisting of:
the metal catalyst is selected from palladium acetate, palladium chloride, cuprous iodide, copper acetate, ferric chloride, ferric acetylacetonate, cobalt chloride, cobalt acetylacetonate, nickel acetate, nickel chloride, or combinations thereof;
the ligand is selected from triphenylphosphine, tricyclohexylphosphine, tri tert -butylphosphine, L-proline, alanine, methionine, pyridine, 2,2-bipyridine, 1,10-phenanthroline, or derivatives thereof;
the base is selected from potassium carbonate, sodium carbonate, potassium phosphate, lithium tert-butoxide, sodium hydroxide, sodium bicarbonate, or combinations thereof;
the solvent is selected from tetrahydrofuran, toluene, acetonitrile, N,N-dimethylformamide, N,N-dimethylacetamide, N-methylpyrrolidone, dimethyl sulfoxide, or combinations thereof.

25. The preparation method according to claim 21, wherein,
R₂ in the compound of formula II is acetyl, the metal catalyst is palladium chloride, the ligand is triphenylphosphine, and the base is potassium carbonate, or
R₂ in the compound of formula II is propionyl, the metal catalyst is nickel acetate, the ligand is 2-bipyridine, and the base is potassium phosphate, or
R₂ in the compound of formula II is N,N-dimethylformamide, the metal catalyst is cuprous iodide, the ligand is 1,10-phenanthroline, and the base is sodium bicarbonate, or
R₂ in the compound of formula II is benzoyl, the metal catalyst is nickel chloride, the ligand is tricyclohexylphosphine, and the base is lithium tert butoxide.

26. The preparation method according to claim 21, wherein in step (1), the reaction temperature is 40-130 °C, preferably 60-115 °C.

27. The preparation method according to claim 21, wherein the method further comprises the following steps:
(1-a) reacting the compound of formula IV with an acylation reagent to obtain the compound of formula II;
the reaction formula is as follows: 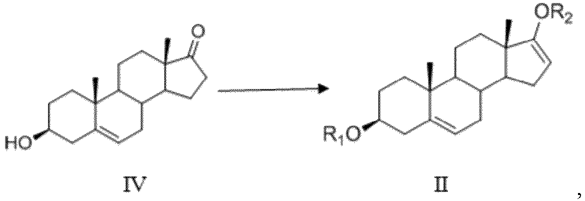
wherein, the acylation reagent used to obtain R₁ is selected from R₁-halogen, R₁-O-R₁ or 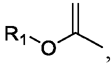 and the acylation reagent used to obtain R₂ is selected from R₂-halogen, R₂-O-R₂ or

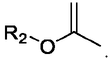

28. The preparation method according to claim 27, wherein the acylation reagent used to obtain R₁ is selected from C₁-C₆ alkyl acyl chloride, substituted or unsubstituted benzoic anhydride, isopropenyl acetate, or trimethylchlorosilane,
the acylation reagent used to obtain R₂ is selected from isopropenyl acetate or substituted or unsubstituted benzoic anhydride.

29. A preparation method for abiraterone, wherein the preparation method comprises deprotecting the compound of formula I prepared by any one of claims 21 to 28 to obtain abiraterone, and the reaction formula is as follows:

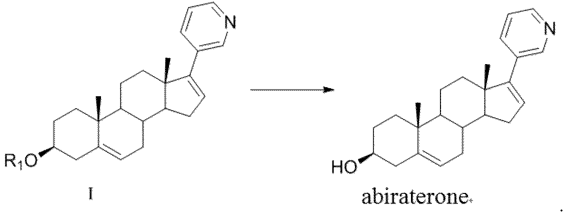

30. A preparation method for abiraterone acetate, wherein the preparation method comprises the steps of: (3) reacting abiraterone prepared by the preparation method according to claim 29 with acetic anhydride to obtain abiraterone acetate, and the reaction formula is as follows:

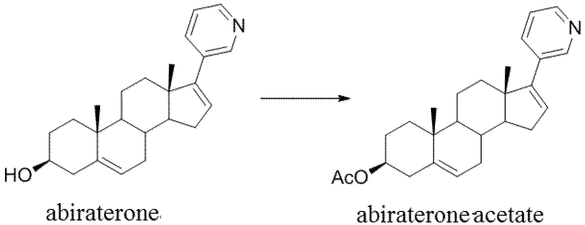

31. A preparation method for abiraterone acetate or an intermediate thereof, wherein the method comprises the steps of: (1) in an organic solvent, in the presence of a metal catalyst, reacting a compound of formula II with a compound of formula III-2 to obtain a compound of formula I;
the reaction formula is as follows: 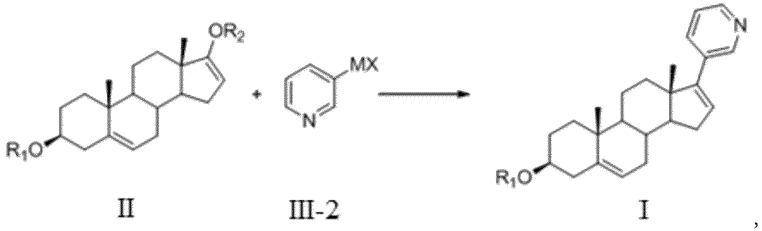
R₁ is selected from hydrogen, C₁-C₆ alkyl, phenyl, benzyl, C₁-C₆ acyl, substituted or unsubstituted benzoyl, methylsulfonyl, substituted or unsubstituted benzenesulfonyl, trialkylsilyl or dialkylarylsilyl, and the alkyl in trialkylsilyl and dialkylarylsilyl is a C₁-C₃ alkyl,
R₂ is selected from C₁-C₆ acyl, substituted or unsubstituted benzoyl, methylsulfonyl, substituted or unsubstituted benzenesulfonyl, dimethylamino acyl, diethylamino acyl, or diphenylphosphinyl acyl,
the term "substituted" refers to that the one or more hydrogen atoms on a group are independently substituted by a group selected from the group consisting of:
halogen, C₁-C₄ alkyl, C₁-C₄ haloalkyl, ether, nitro,
M is magnesium, iron, zinc, copper, tin, manganese, bismuth, or indium,
X is fluorine, chlorine, bromine, iodine, or a coordinated anion of M, preferably, the coordinated anion of M is selected from pivalate or acetate,
the metal catalyst is selected from palladium salt, copper salt, iron salt, cobalt salt, nickel salt, or combinations thereof.

32. The preparation method according to claim 31, wherein a ligand and/or additive may be present in the reaction system,
the ligand is selected from a phosphorus-containing ligand, amino acid ligand, nitrogen-containing ligand, or combinations thereof,
the additive is selected from alkali metal salt.

33. The preparation method according to claim 31 or 32, wherein R₁ is selected from C₁-C₆ acyl, substituted or unsubstituted benzoyl, trialkylsilyl, more preferably acetyl, and/or
R₂ is selected from C₁-C₆ acyl, substituted or unsubstituted benzoyl, more preferably acetyl, and/or
M is magnesium, zinc, tin, or manganese, and/or
X is chlorine or bromine.

34. The method according to claim 31 or 32, wherein step (1) has one or more features selected from the group consisting of:
the metal catalyst is selected from bis triphenylphosphine palladium chloride, palladium acetate, palladium chloride, cuprous iodide, copper acetate, copper sulfate, ferric chloride, ferrous chloride, ferric acetylacetonate, ferrous acetylacetonate, cobalt chloride, cobalt acetate, acetylacetonate cobalt, nickel chloride, nickel bromide, nickel acetate, tricyclohexylphosphine nickel chloride, or combinations thereof,
the organic solvent is selected from tetrahydrofuran, 2-methyltetrahydrofuran, ethylene glycol dimethyl ether, methyl tert-butyl ether, ether, toluene, or combinations thereof,
the molar ratio of the compound of formula II to the compound of formula III-2 is 1: 1.0~4.0, preferably 1: 1.0~2.0,
the molar ratio of the compound of formula II to the metal catalyst is 1: 0.005~0.3; preferably, 1: 0.01~0.2,
the temperature for addition of the compound of III-2 is -30 °C~25 °C, and the reaction temperature is 0~70 °C, preferably room temperature ~50 °C.

35. The method according to claim 32, wherein,
the ligand is selected from triphenylphosphine, tricyclohexylphosphine, tri tert-butylphosphine, L-proline, alanine, methionine, pyridine, 2,2-bipyridine, 1,10-phenanthroline or derivatives thereof, and/or
the molar ratio of the compound of formula II to the ligand is 1: 0.005~0.3; preferably, 1: 0.01~0.2, and/or
the additive is selected from potassium chloride, potassium carbonate, potassium tert-butoxide, potassium bicarbonate, potassium phosphate, potassium acetate, sodium carbonate, sodium bicarbonate, sodium acetate, lithium chloride, lithium carbonate, or combinations thereof, and/or
the molar ratio of the compound of formula II to the additive is 1: 0.1~3; more preferably 1: 1~2.

36. The method according to claim 32, wherein,
in the compound of formula II, R₂ is acetyl, the compound of III-2 is 3-pyridinium magnesium bromide, the metal catalyst is PdCl₂, the ligand is triphenylphosphine, and the additive is sodium acetate, or
in the compound of formula II, R₂ is acetyl, the compound of III-2 is 3-pyridylzinc bromide, the metal catalyst is anhydrous ferric chloride, the ligand is tetramethylethylenediamine, and the additive is potassium acetate, or
in the compound of formula II, R₂ is p-phenylsulfonyl, the compound of III-2 is 3-pyridinium tin bromide, the metal catalyst is nickel acetate, the ligand is tricyclohexylphosphine, and the additive is sodium carbonate, or
in the compound of formula II, R₂ is benzoyl, the compound of formula III-2 is 3-pyridinium manganese bromide, the metal catalyst is cuprous iodide, the ligand is L-proline, and the additive is anhydrous potassium phosphate.

37. The preparation method according to claim 31 or 32, wherein the method further comprises the following steps:
(1-a) reacting the compound of formula IV with an acylation reagent to obtain the compound of formula II;
the reaction formula is as follows: 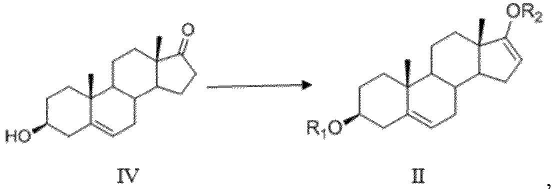
wherein, the acylation reagent used to obtain R₁ is selected from R₁-halogen, R₁-O-R₁ or 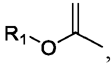 and the acylation reagent used to obtain R₂ is selected from R₂-halogen, R₂-O-R₂ or

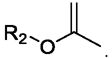

38. The preparation method according to claim 37, wherein the acylation reagent used to obtain R₁ is selected from C₁-C₆ alkyl acyl chloride, substituted or unsubstituted benzoic anhydride, isopropenyl acetate, or trimethylchlorosilane, and/or
the acylation reagent used to obtain R₂ is selected from isopropenyl acetate or substituted or unsubstituted benzoic anhydride.

39. A preparation method for abiraterone, wherein the preparation method comprises the steps of: (2) deprotecting the compound of formula I prepared by the preparation method according to any one of claims 31 to 38 to obtain abiraterone, and the reaction formula is as follows:

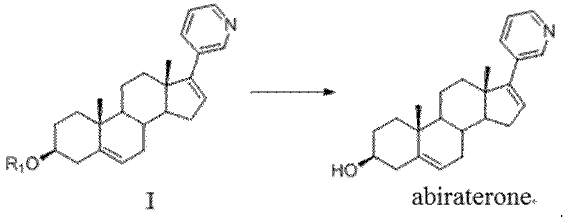

40. A preparation method for abiraterone acetate, wherein the preparation method comprises the steps of: (3) reacting abiraterone prepared by the preparation method according to claim 39 with acetic anhydride to obtain abiraterone acetate, the reaction formula is as follows:
